# EUROPEAN PATENT SPECIFICATION

(11) **EP 4 190 815 B1**
(45) Date of publication and mention of the grant of the patent: **06.05.2026**
(21) Application number: 22766246.7
(22) Date of filing: 07.03.2022
(51) Int. Cl.: C07K 16/28, C12N 15/13, A61P 35/00, A61K 39/395, G01N 33/574

(54) **ANTI-PVRIG PROTEIN ANTIBODY OR ANTIBODY FRAGMENT, AND USE THEREOF**
ANTI-PVRIG-PROTEINANTIKÖRPER ODER ANTIKÖRPERFRAGMENT UND VERWENDUNG DAVON
ANTICORPS DE PROTÉINE ANTI-PVRIG OU FRAGMENT D'ANTICORPS, ET SON UTILISATION

(30) Priority: 08.03.2021 CN 202110250342
(43) Date of publication of application: 07.06.2023
(73) Proprietor: Hefei TG Immunopharma Co., Ltd., Hefei, Anhui 230001 (CN)
(72) Inventor: TIAN, Zhigang, Hefei, Anhui 230001 (CN); LI, Yangyang, Hefei, Anhui 230001 (CN); XIAO, Weihua, Hefei, Anhui 230001 (CN); SUN, Rui, Hefei, Anhui 230001 (CN); SUN, Haoyu, Hefei, Anhui 230001 (CN)
(74) Representative: Wilson, Justin Scott
(86) International application number: PCT/CN2022/079449
(87) International publication number: WO 2022/188721

(56) References cited:
- WO-A2-2018/017864
- CN-A- 107 580 500
- CN-A- 110 088 132
- CN-A- 112 433 055
- US-A1- 2017 088 607

## Description

The present application claims priority to Chinese Patent Application No. 202110250342.9, filed on March 08, 2021.

### TECHNICAL FIELD

The present disclosure relates to the field of pharmaceuticals, and more particularly to an anti-PVRIG protein antibody or antibody fragment and use thereof in cancer therapy.

### BACKGROUND

Tumor cells evade surveillance of the immune system through a variety of mechanisms. The immune checkpoint pathway is used for the maintenance of self-tolerance and control of activated lymphocyte effector function, but cancer cells can utilize this pathway to avoid disruption. Recent reports indicate that PVRIG is a very important immune checkpoint. PVRIG is mainly expressed on activated T cells and NK cells and inhibits the effector function of T cells and NK cells by interacting with the ligand CD112 expressed on target cells or DC cells. CD112 is highly expressed on the surface of many kinds of tumor cells, so as to inhibit the function of the immune system through PVRIG signaling pathway and realize immune escape.

It has been shown in researches that deletion of PVRIG may result in enhanced effector function of CD8+ T cells. In addition, the use of antibodies that block the binding of PVRIG to its ligand CD 112 can also be effective in restoring the function of tumor-infiltrating CD8+ T cells and inhibiting tumor growth. An antibody targeting human PVRIG has been in clinical trials, showing good therapeutic effects. CN107580500A is directed to anti-PVRIG antibodies and methods of using them. CN112433055A discloses a method for detecting the biological activity of a PVRIG antibody based on a reporter gene method. CN110088132A discloses anti-TIGIT antibodies, anti-PVRIG antibodies and combinations thereof. WO2018/017864A2 discloses agents that specifically bind PVRIG. US2017/088607A1 is directed to PVRIG polypeptides and their uses.

Further improvements are needed for antibodies that target PVRIG.

### SUMMARY

The present disclosure aims to provide an antibody or an antibody fragment with an enhanced anti-tumor function and use thereof. The provided antibody or antibody fragment is capable of effectively binding to PVRIG and is capable of blocking the binding of PVRIG to its ligand CD112. The provided antibody or antibody fragment have been verified to have high affinity to human PVRIG, and is effective in blocking the interaction between human PVRIG and CD112.

In a first aspect, the present disclosure provides an antibody or antibody fragment including a heavy chain variable region and a light chain variable region, the heavy chain variable region being selected from the group consisting of sequences set forth in SEQ ID NO: 9, SEQ ID NO: 15, SEQ ID NO: 18, SEQ ID NO: 21, SEQ ID NO: 24, SEQ ID NO: 27, SEQ ID NO: 30, and SEQ ID NO: 33, and the light chain variable region being a sequence set forth in SEQ ID NO: 36.

In a second aspect, the present disclosure provides an isolatable nucleic acid encoding any of antibodies or antibody fragments as described above.

In a third aspect, the present disclosure provides an expression vector including the nucleic acid as described above.

In a fourth aspect, the present disclosure provides a recombinant cell expressing any of antibodies or antibody fragments as described above.

In a fifth aspect, the present disclosure provides a pharmaceutical composition including the antibody or antibody fragment as described above and a pharmaceutically acceptable carrier.

In a sixth aspect, the present disclosure provides a pharmaceutical combination including: (1) the antibody or antibody fragment as described above, or the pharmaceutical composition as described above; and (2) an antibody for cancer therapy other than (1).

In a seventh aspect, the present disclosure provides the antibody or antibody fragment as described above or the pharmaceutical composition as described above for use in preventing or treating cancer.

In an eighth aspect, the present disclosure provides a method for inhibiting PVRIG protein activity in a sample in vitro, including contacting the sample with any of antibodies or antibody fragments as described above, or the pharmaceutical composition as described above. According to an embodiment of the present disclosure, the sample may be a cell sample.

The antibody or antibody fragment provided by the present disclosure is capable of specifically bind to the PVRIG antigen and greatly enhancing the killing effect of human PBMC and exhibits good anti-tumor effects in mouse models. Additional features and advantages of the present disclosure will be set forth in the detailed description which follows.

### BRIEF DESCRIPTION OF DRAWINGS

FIG. 1 is a graph showing the ELISA analysis results of binding of the murine antibody to human PVRIG according to an embodiment of the present disclosure;
FIG. 2 is a graph showing the analysis results of binding of the murine antibody to human PVRIG on the surface of a cell membrane according to an embodiment of the present disclosure;
FIG. 3 is a graph showing the analysis results of blocking the binding of human PVRIG to human CD112 by the murine antibody according to an embodiment of the present disclosure;
FIG. 4 is a graph showing the analysis results of competitive binding of the murine antibody to human PVRIG on the surface of a cell membrane according to an embodiment of the present disclosure;
FIG. 5 is a graph showing the activity results of binding of murine antibodies to Macaca fascicularis PVRIG and mouse PVRIG on the cell membrane surface according to an embodiment of the present disclosure;
FIG. 6 is a graph showing the ELISA analysis results of binding of the murine antibody to Macaca fascicularis PVRIG according to an embodiment of the present disclosure;
FIG. 7 is a graph showing the activity results of the murine antibody in detecting PVRIG on the membrane surface of a NKG cell line according to an embodiment of the present disclosure;
FIG. 8 is a graph showing the activity results of the murine antibody in detecting PVRIG on the surface of lymphocytes according to an embodiment of the present disclosure;
FIG. 9 is a graph showing the results of CD112 expression on the surface of a human tumor cell line according to an embodiment of the present disclosure;
FIG. 10 is a graph showing the results of promoting cytotoxicity of NK cells by the murine antibody according to an embodiment of the present disclosure;
FIG. 11 is a graph showing the results of promoting cytotoxicity of human PBMC by the murine antibody according to an embodiment of the present disclosure;
FIG. 12 is a graph showing the results of promoting the expression of effector molecules of NK cells by the murine antibody according to an embodiment of the present disclosure;
FIG. 13 is a graph showing the ELISA analysis results of promoting the secretion of IFN-γ from NK cells by the murine antibody according to an embodiment of the present disclosure;
FIG. 14 is a graph of the analysis results of inhibiting tumor growth in vivo by the murine antibody according to an embodiment of the present disclosure;
FIG. 15 is a graph showing the ELISA analysis results of binding of the humanized antibody to human PVRIG according to an embodiment of the present disclosure;
FIG. 16 is a graph showing the analysis results of binding of the humanized antibody to human PVRIG on the cell membrane surface according to an embodiment of the present disclosure;
FIG. 17 is a graph showing the analysis results of binding of the humanized antibody to Macaca fascicularis PVRIG on the cell membrane surface according to an embodiment of the present disclosure;
FIG. 18 is a graph of the analysis results of blocking the binding of human PVRIG to human CD112 by the humanized antibody according to an embodiment of the present disclosure; and
FIG. 19 is a graph showing the results of promoting the cytotoxicity of human PBMC by the humanized antibody according to an embodiment of the present disclosure.

### DESCRIPTION OF EMBODIMENTS

The embodiments of the present disclosure will be described in detail below, and are intended to explain the present disclosure and are not to be construed as limiting the present disclosure. In addition, some terms herein have been described and interpreted to facilitate understanding by those skilled in the art and should not be construed as limiting the scope of the present disclosure.

As used herein, the term "antibody" refers to a protein that is capable of interacting with an antigen (e.g., by binding, steric hindrance, stabilizing spatial distribution) and includes two heavy chains and two light chains linked by disulfide bonds. Each heavy chain includes a heavy chain variable region (abbreviated as VH) and a heavy chain constant region. The heavy chain constant region includes three domains CH1, CH2, and CH3. Each light chain includes a light chain variable region (abbreviated as VL general) and a light chain constant region. The light chain constant region includes one domain CL. The heavy chain variable region and the light chain variable region each may be further subdivided into complementary determining regions (CDRs). Each heavy chain variable region and each light chain variable region have three CDR regions, CDR1, CDR2, and CDR3 from the amino terminus to the carboxyl terminus. As used herein, the three CDR regions of the heavy chain are designated as HCDR1, HCDR2, and HCDR3; the three CDR regions of the light chain are designated as LCDR1, LCDR2, and LCDR3.

The antibody in the expression "antibody or antibody fragment" referred to herein includes, but is not limited to, monoclonal antibodies, human antibodies, humanized antibodies, camelised antibodies, and chimeric antibodies. The antibody can be of any isotype, e.g., IgG, IgE, IgM, IgD, IgA, and IgY, etc.

The term "antibody fragment" refers to one or more sections of an antibody that retain the ability to specifically interact with an antigen (e.g., by binding, steric hindrance, stabilizing spatial distribution). Examples of the antibody fragment include, but are not limited to, a Fab fragment, a monovalent fragment consisting of the VL, VH, CL, and CH1 domains; a F(ab)₂ fragment, a bivalent fragment including two Fab fragments linked by a disulfide bridge at the hinge region; a Fd fragment consisting of the VH and CH1 domains; a Fv fragment consisting of the VL and VH domains of a single arm of an antibody; and isolated CDRs. Furthermore, although the two domains of the Fv fragment, VL and VH, are encoded by separate genes, they can be joined using recombinant methods by a synthetic linking moiety that enables them to form a single protein chain, where the VL and VH regions pair to form a monovalent molecule (referred to as a single chain Fv (scFv); see, e.g., Bird et al., (1988) Science 242:423-426; and Huston et al., (1988) Proc. Natl. Acad. Sci. 85:5879-5883). Such single chain antibodies are also encompassed by the term "antibody fragment". These antibody fragments are obtained using conventional techniques known to those skilled in the art, and fragments having applicability in the same manner as the complete antibody are selected.

The "humanized antibody" refers to: (i) a non-human source derived antibody (e.g., a transgenic mouse carrying a heterologous immune system) based on human germline sequences, or (ii) a CDR-grafted antibody, the CDR of the variable domain of which is non-human derived, and one or more frameworks of the variable domain of which are human derived, and the constant domain of which, if present, is human derived.

The term "chimeric antibody" refers to an antibody having a constant antibody region derived from or corresponding to a sequence found in one species and a variable antibody region derived from another species. Preferably, the constant antibody regions are derived from or correspond to sequences found in humans, e.g., in human germline cells or somatic cells, and the variable antibody regions (e.g., VH, VL, CDR, or FR regions) are derived from sequences found in non-human animals, e.g., mice, rats, rabbits, or hamsters.

As used herein, the term "recombinant antibody" includes all antibodies prepared, expressed, produced, or isolated by recombination manners, for example, antibodies isolated from human immunoglobulin gene transgenic or transchromosomal animals (e.g., mice) or hybridomas prepared therefrom, antibodies isolated from host cells transformed to express the antibodies, antibodies selected and isolated from recombinant, combinatorial human antibody libraries, and antibodies prepared, expressed, produced, or isolated by any other means involving the splicing of part or all of the human immunoglobulin gene and sequence to other DNA sequences. Preferably, these recombinant antibodies have variable regions in which the framework and CDR regions are derived from human germline immunoglobulin sequences.

As used herein, the term "monoclonal antibody" refers to an antibody molecule preparation consisting of a single molecule. Monoclonal antibodies exhibit unique binding sites that have unique binding specificity and affinity to a particular epitope.

The sequences of the provided antibody or antibody fragment were subjected to humanization modification, and the humanized antibody or antibody fragments are shown below.

In at least some embodiments, the antibody or antibody fragment includes a heavy chain variable region and a light chain variable region, the heavy chain variable region is a sequence set forth in SEQ ID NO: 9 or a sequence having at least one amino acid substitution compared to the sequence set forth in SEQ ID NO: 9, and the light chain variable region is a sequence set forth in SEQ ID NO: 36 or a sequence having at least one amino acid substitution compared to the sequence set forth in SEQ ID NO: 36.

In at least some preferred embodiments, the antibody is Antibody 4-hu3. A heavy chain variable region of Antibody 4-hu3 is a sequence set forth in SEQ ID NO: 9, and a light chain variable region of Antibody 4-hu3 is a sequence set forth in SEQ ID NO: 36. In at least some preferred embodiments, an amino acid sequence of the heavy chain of Antibody 4-hu3 is set forth in SEQ ID NO: 10, and the corresponding nucleotide sequence encoding the amino acid sequence of the heavy chain is set forth in SEQ ID NO: 11; an amino acid sequence of the light chain of Antibody 4-hu3 is set forth in SEQ ID NO: 37, and the corresponding nucleotide sequence encoding the amino acid sequence of the light chain is set forth in SEQ ID NO: 38.

In at least some embodiments, the antibody or antibody fragment includes a heavy chain variable region and a light chain variable region, the heavy chain variable region is a sequence set forth in SEQ ID NO: 15 or a sequence having at least one amino acid substitution compared to the sequence set forth in SEQ ID NO: 15, and the light chain variable region is a sequence set forth in SEQ ID NO: 36 or a sequence having at least one amino acid substitution compared to the sequence set forth in SEQ ID NO: 36.

In at least some preferred embodiments, the antibody is Antibody 4-hu5. A heavy chain variable region of Antibody 4-hu5 is a sequence set forth in SEQ ID NO: 15, and a light chain variable region of Antibody 4-hu5 is a sequence set forth in SEQ ID NO: 36. In at least some preferred embodiments, an amino acid sequence of the heavy chain of Antibody 4-hu5 is set forth in SEQ ID NO: 16, and the corresponding nucleotide sequence encoding the amino acid sequence of the heavy chain is set forth in SEQ ID NO: 17; an amino acid sequence of the light chain of Antibody 4-hu5 is set forth in SEQ ID NO: 37, and the corresponding nucleotide sequence encoding the amino acid sequence of the light chain is set forth in SEQ ID NO: 38.

In at least some embodiments, the antibody or antibody fragment includes a heavy chain variable region and a light chain variable region, the heavy chain variable region is a sequence set forth in SEQ ID NO: 18 or a sequence having at least one amino acid substitution compared to the sequence set forth in SEQ ID NO: 18, and the light chain variable region is a sequence set forth in SEQ ID NO: 36 or a sequence having at least one amino acid substitution compared to the sequence set forth in SEQ ID NO: 36.

In at least some preferred embodiments, the antibody is Antibody 4-hu6. A heavy chain variable region of Antibody 4-hu6 is a sequence set forth in SEQ ID NO: 18, and a light chain variable region of Antibody 4-hu6 is a sequence set forth in SEQ ID NO: 36. In at least some preferred embodiments, an amino acid sequence of the heavy chain of Antibody 4-hu6 is set forth in SEQ ID NO: 19, and the corresponding nucleotide sequence encoding the amino acid sequence of the heavy chain is set forth in SEQ ID NO: 20; an amino acid sequence of the light chain of Antibody 4-hu6 is set forth in SEQ ID NO: 37, and the corresponding nucleotide sequence encoding the amino acid sequence of the light chain is set forth in SEQ ID NO: 38.

In at least some embodiments, the antibody or antibody fragment includes a heavy chain variable region and a light chain variable region, the heavy chain variable region is a sequence set forth in SEQ ID NO: 21 or a sequence having at least one amino acid substitution compared to the sequence set forth in SEQ ID NO: 21, and the light chain variable region is a sequence set forth in SEQ ID NO: 36 or a sequence having at least one amino acid substitution compared to the sequence set forth in SEQ ID NO: 36.

In at least some preferred embodiments, the antibody is Antibody 4-hu7. A heavy chain variable region of Antibody 4-hu7 is a sequence set forth in SEQ ID NO: 21, and a light chain variable region of Antibody 4-hu7 is a sequence set forth in SEQ ID NO: 36. In at least some preferred embodiments, an amino acid sequence of the heavy chain of Antibody 4-hu7 is set forth in SEQ ID NO: 22, and the corresponding nucleotide sequence encoding the amino acid sequence of the heavy chain is set forth in SEQ ID NO: 23; an amino acid sequence of the light chain of Antibody 4-hu7 is set forth in SEQ ID NO: 37, and the corresponding nucleotide sequence encoding the amino acid sequence of the light chain is set forth in SEQ ID NO: 38.

In at least some embodiments, the antibody or antibody fragment includes a heavy chain variable region and a light chain variable region, the heavy chain variable region is a sequence set forth in SEQ ID NO: 24 or a sequence having at least one amino acid substitution compared to the sequence set forth in SEQ ID NO: 24, and the light chain variable region is a sequence set forth in SEQ ID NO: 36 or a sequence having at least one amino acid substitution compared to the sequence set forth in SEQ ID NO: 36.

In at least some preferred embodiments, the antibody or antibody fragment is Antibody 4-hu8. A heavy chain variable region of Antibody 4-hu8 is a sequence set forth in SEQ ID NO: 24, and a light chain variable region of Antibody 4-hu8 is a sequence set forth in SEQ ID NO: 36. In at least some preferred embodiments, an amino acid sequence of the heavy chain of Antibody 4-hu8 is set forth in SEQ ID NO: 25, and the corresponding nucleotide sequence encoding the amino acid sequence of the heavy chain is set forth in SEQ ID NO: 26; an amino acid sequence of the light chain of Antibody 4-hu8 is set forth in SEQ ID NO: 37, and the corresponding nucleotide sequence encoding the amino acid sequence of the light chain is set forth in SEQ ID NO: 38.

In at least some embodiments, the antibody or antibody fragment includes a heavy chain variable region and a light chain variable region, the heavy chain variable region is a sequence set forth in SEQ ID NO: 27 or a sequence having at least one amino acid substitution compared to the sequence set forth in SEQ ID NO: 27, and the light chain variable region is a sequence set forth in SEQ ID NO: 36 or a sequence having at least one amino acid substitution compared to the sequence set forth in SEQ ID NO: 36.

In at least some preferred embodiments, the antibody is Antibody 4-hu9. A heavy chain variable region of Antibody 4-hu9 is a sequence set forth in SEQ ID NO: 27, and a light chain variable region of Antibody 4-hu9 is a sequence set forth in SEQ ID NO: 36. In at least some preferred embodiments, an amino acid sequence of the heavy chain of Antibody 4-hu9 is set forth in SEQ ID NO: 28, and the corresponding nucleotide sequence encoding the amino acid sequence of the heavy chain is set forth in SEQ ID NO: 29; an amino acid sequence of the light chain of Antibody 4-hu9 is set forth in SEQ ID NO: 37, and the corresponding nucleotide sequence encoding the amino acid sequence of the light chain is set forth in SEQ ID NO: 38.

In at least some embodiments, the antibody or antibody fragment includes a heavy chain variable region and a light chain variable region, the heavy chain variable region is a sequence set forth in SEQ ID NO: 30 or a sequence having at least one amino acid substitution compared to the sequence set forth in SEQ ID NO: 30, and the light chain variable region is a sequence set forth in SEQ ID NO: 36 or a sequence having at least one amino acid substitution compared to the sequence set forth in SEQ ID NO: 36.

In at least some preferred embodiments, the antibody is Antibody 4-hu10. A heavy chain variable region of Antibody 4-hu10 is a sequence set forth in SEQ ID NO: 30, and a light chain variable region of Antibody 4-hu10 is a sequence set forth in SEQ ID NO: 36. In at least some preferred embodiments, an amino acid sequence of the heavy chain of Antibody 4-hu10 is set forth in SEQ ID NO: 31, and the corresponding nucleotide sequence encoding the amino acid sequence of the heavy chain is set forth in SEQ ID NO: 32; an amino acid sequence of the light chain of Antibody 4-hu10 is set forth in SEQ ID NO: 37, and the corresponding nucleotide sequence encoding the amino acid sequence of the light chain is set forth in SEQ ID NO: 38.

In at least some embodiments, the antibody or antibody fragment includes a heavy chain variable region and a light chain variable region, the heavy chain variable region is a sequence set forth in SEQ ID NO: 33 or a sequence having at least one amino acid substitution compared to the sequence set forth in SEQ ID NO: 33, and the light chain variable region is a sequence set forth in SEQ ID NO: 36 or a sequence having at least one amino acid substitution compared to the sequence set forth in SEQ ID NO: 36.

In at least some preferred embodiments, the antibody is Antibody 4-hu11, A heavy chain variable region of Antibody 4-hu11 is a sequence set forth in SEQ ID NO: 33, and a light chain variable region of Antibody 4-hu11 is a sequence set forth in SEQ ID NO: 36. In at least some preferred embodiments, an amino acid sequence of the heavy chain of Antibody 4-hu11 is set forth in SEQ ID NO: 34, and the corresponding nucleotide sequence encoding the amino acid sequence of the heavy chain is set forth in SEQ ID NO: 35; an amino acid sequence of the light chain of Antibody 4-hu11 is set forth in SEQ ID NO: 37, and the corresponding nucleotide sequence encoding the amino acid sequence of the light chain is set forth in SEQ ID NO: 38.

The nucleic acid sequence encoding the amino acid sequence of the heavy chain of Antibody 4-hu1 is set forth in SEQ ID NO: 5:

GAGGTGCAGCTGGTGCAGTCCGGCGCTGAGGTGAAGAAGCCCGGCTCCTCCGTG
AAGGTGAGCTGCAAGGCTTCCGGCGGCACCTTTTCCAGCTTCTCCATCGAGTGGG
TGAGGCAGGCCCCCGGCCAGGGATTGGAGTGGATGGGCGAGATCCTGCCTGGCTC
CAACAGCACCAACTACAATGAGAAGTTTAAGGGCAGGGTGACCATCACCGCTGAC
GAGTCCACCTCCACCGCCTACATGGAGCTGTCCTCCCTGCGGTCCGAGGATACCGC
TGTGTACTATTGTGCCAGGTATTGGTTCGCTTATTGGGGCCAGGGCACCCTGGTGA
CCGTGTCCTCCGCCTCCACCAAGGGCCCCTCCGTGTTCCCCCTGGCTCCCTGTAGC
CGGTCCACCAGCGAGTCCACCGCTGCCCTGGGCTGTCTGGTGAAGGACTATTTCC
CCGAGCCCGTGACCGTGAGCTGGAATAGCGGCGCCCTGACCTCCGGCGTGCACAC
ATTCCCTGCTGTGCTGCAGAGCTCCGGCCTGTATAGCCTGTCCTCCGTGGTGACCG
TGCCCTCCTCCTCCCTGGGCACCAAGACCTATACCTGCAATGTGGACCACAAGCCC
AGCAACACCAAGGTGGACAAGAGGGTGGAGAGCAAGTACGGCCCTCCTTGCCCT
CCTTGCCCCGCTCCAGAGTTCCTGGGCGGCCCAAGCGTGTTTCTGTTTCCTCCCAA
GCCTAAGGACACCCTGATGATCTCCAGGACCCCTGAGGTGACCTGCGTGGTGGTG

GATGTGTCCCAGGAGGACCCTGAGGTGCAGTTCAACTGGTACGTGGATGGCGTGG
AGGTGCACAACGCCAAGACCAAGCCCCGGGAGGAGCAGTTCAACAGCACCTACC
GGGTGGTGAGCGTGCTGACCGTGCTGCACCAGGATTGGCTGAATGGCAAGGAGTA
CAAGTGCAAGGTGTCCAACAAGGGCCTGCCTAGCAGCATCGAGAAGACCATCTCC
AAGGCCAAGGGCCAGCCCAGGGAGCCTCAGGTGTATACCCTGCCCCCTAGCCAGG
AGGAGATGACCAAGAACCAGGTGAGCCTGACCTGCCTGGTGAAGGGCTTTTATCC
TAGCGATATCGCTGTGGAGTGGGAGTCCAACGGCCAGCCCGAGAACAACTACAAG
ACCACCCCTCCCGTGCTGGATAGCGACGGCTCCTTCTTTCTGTACTCCCGGCTGAC
CGTGGATAAGTCCAGGTGGCAGGAGGGCAATGTGTTCAGCTGCTCCGTGATGCAC
GAGGCCCTGCACAATCACTATACCCAGAAGTCCCTGTCCCTGTCCCTCGGCAAG
(SEQ ID NO: 5).

The nucleic acid sequence encoding the amino acid sequence of the heavy chain of Antibody 4-hu2 is set forth in SEQ ID NO: 8:

GAGGTGCAGCTGGTGCAGAGCGGCGCTGAGGTGAAGAAGCCTGGCAGCTCCGTG
AAGGTGAGCTGCAAGGCCTCCGGCGGCACCTTCTCCAGCTTTTCCATCGAGTGGG
TGAGGCAGGCCCCTGGCCAGGGACTGGAGTGGATGGGCGAGATCCTGCCTGGCA
GCAATTCCACCAACTATAATGAGAAGTTCAAGGGCCGGGTGACCATCACCGCTGA
CGAGAGCACCTCCACCGCTTACATGGAGCTGTCCAGCCTGAGGTCCGAGGACACC
GCTGTGTACTACTGTAGCTCCTATTGGTTCGCTTACTGGGGCCAGGGCACCCTGGT
GACCGTGTCCTCCGCCAGCACCAAGGGCCCTAGCGTGTTCCCCCTGGCTCCCTGC
AGCCGGAGCACCTCTGAGTCCACCGCCGCCCTGGGCTGTCTGGTGAAGGATTACT
TTCCTGAGCCCGTGACCGTGAGCTGGAATAGCGGCGCTCTGACCAGCGGCGTGCA
CACCTTTCCTGCCGTGCTGCAGAGCAGCGGCCTGTACAGCCTGTCCTCCGTGGTG
ACCGTGCCTAGCTCCAGCCTGGGCACCAAGACCTATACCTGTAATGTGGATCACAA
GCCTAGCAATACCAAGGTGGACAAGCGGGTGGAGTCCAAGTACGGCCCCCCCTGC
CCTCCCTGCCCAGCTCCTGAATTTCTGGGCGGCCCCAGCGTGTTTCTGTTTCCTCC
CAAGCCTAAGGATACCCTGATGATCTCCAGGACCCCCGAGGTGACCTGTGTGGTG
GTGGACGTGTCCCAGGAGGATCCCGAGGTGCAGTTTAACTGGTACGTGGACGGCG
TGGAGGTGCACAATGCCAAGACCAAGCCTAGGGAGGAGCAGTTCAATAGCACCTA
CAGGGTGGTGAGCGTGCTGACCGTGCTGCACCAGGATTGGCTGAATGGCAAGGAG

TATAAGTGCAAGGTGTCCAACAAGGGCCTGCCCTCCAGCATCGAGAAGACCATCA
GCAAGGCCAAGGGCCAGCCCCGGGAGCCTCAGGTTTATACCCTGCCCCCTAGCCA
GGAGGAGATGACCAAGAACCAGGTGTCCCTGACCTGTCTGGTGAAAGGCTTCTAT
CCTTCCGATATCGCCGTGGAGTGGGAGTCCAATGGCCAGCCCGAGAACAATTATAA
GACCACCCCTCCCGTGCTGGATTCCGACGGCAGCTTTTTCCTGTACTCCCGGCTGA
CCGTGGATAAGAGCCGGTGGCAGGAGGGCAATGTGTTTAGCTGCAGCGTGATGCA
CGAGGCTCTGCACAACCACTACACCCAGAAGTCCCTGAGCCTGTCCCTGGGCAAG
(SEQ ID NO: 8).

The amino acid sequence of the heavy chain variable region of Antibody 4-hu3 is set forth in SEQ ID NO: 9:

EVQLVQSGAEVKKPGSSVKVSCKASGGTFSSFSIEWVRQAPGQGLEWMGEILPGSNS
TNYNEKFKGRVTFTADESTSTAYMELSSLRSEDTAVYYCSSYWFAYWGQGTLVTVSS
(SEQ ID NO:9).

The amino acid sequence of the heavy chain of Antibody 4-hu3 is set forth in SEQ ID NO: 10 (IgG4 S228P mutation):

EVQLVQSGAEVKKPGSSVKVSCKASGGTFSSFSIEWVRQAPGQGLEWMGEILPGSNS
TNYNEKFKGRVTFTADESTSTAYMELSSLRSEDTAVYYCSSYWFAYWGQGTLVTVSS
ASTKGPSVFPLAPCSRSTSESTAALGCLVKDYFPEPVTVSWNSGALTSGVHTFPAVLQS
SGLYSLSSVVTVPSSSLGTKTYTCNVDHKPSNTKVDKRVESKYGPPCPPCPAPEFLGGP
SVFLFPPKPKDTLMISRTPEVTCVVVDVSQEDPEVQFNWYVDGVEVHNAKTKPREEQ
FNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLT
VDKSRWQEGNVFSCSVMHEALHNHYTQKSLSLSLGK (SEQ ID NO: 10).

The nucleic acid sequence encoding the amino acid sequence of the heavy chain of Antibody 4-hu3 is set forth in SEQ ID NO: 11:

GAGGTGCAGCTGGTGCAGAGCGGCGCTGAGGTGAAGAAGCCTGGCTCCAGCGTG
AAGGTGAGCTGTAAGGCCAGCGGCGGCACCTTCTCCTCCTTTTCCATCGAGTGGG
TGAGGCAGGCCCCCGGCCAGGGATTGGAGTGGATGGGCGAGATCCTGCCCGGCAG
CAATTCCACCAATTATAATGAGAAGTTTAAGGGCCGGGTGACCTTTACCGCCGACG
AGTCCACCTCCACCGCCTATATGGAGCTGAGCTCCCTGAGGAGCGAGGACACCGC

CGTGTACTATTGCAGCTCCTATTGGTTTGCTTATTGGGGCCAGGGCACCCTGGTGAC
CGTGTCCAGCGCCAGCACCAAGGGCCCCTCCGTGTTTCCCCTGGCTCCCTGTAGCC
GGAGCACCAGCGAGTCCACCGCTGCTCTGGGCTGTCTGGTGAAGGATTACTTTCC
CGAGCCCGTGACCGTGTCTTGGAATAGCGGCGCCCTGACCAGCGGCGTGCACACA
TTTCCCGCTGTGCTGCAGAGCTCCGGCCTGTACTCCCTGTCCTCCGTGGTGACCGT
GCCTTCCAGCTCCCTGGGCACCAAGACCTATACCTGCAACGTGGATCACAAGCCTA
GCAACACCAAGGTGGACAAGCGGGTGGAGAGCAAGTACGGCCCCCCCTGCCCCC
CTTGTCCTGCTCCTGAGTTCCTGGGCGGCCCTTCCGTGTTTCTGTTTCCTCCCAAGC
CCAAGGATACCCTGATGATCAGCAGGACCCCTGAGGTGACCTGTGTGGTGGTGGA
CGTGTCCCAGGAGGATCCTGAGGTGCAGTTCAATTGGTATGTGGACGGCGTGGAG
GTGCACAACGCCAAGACCAAGCCCCGGGAGGAGCAGTTCAATTCCACCTATCGGG
TGGTGAGCGTGCTGACCGTGCTGCACCAGGACTGGCTGAACGGCAAGGAGTACA
AGTGCAAGGTGAGCAATAAGGGCCTGCCTAGCTCCATCGAGAAGACCATCTCCAA
GGCCAAGGGCCAGCCTCGGGAGCCCCAGGTTTATACCCTGCCCCCCTCCCAGGAG
GAGATGACCAAGAACCAGGTGAGCCTGACCTGTCTGGTGAAAGGCTTCTACCCCA
GCGATATCGCTGTGGAGTGGGAGTCCAACGGCCAGCCCGAGAATAACTATAAGAC
CACCCCCCCTGTGCTGGATAGCGATGGCAGCTTCTTCCTGTACTCCAGGCTGACCG
TGGACAAGTCCAGGTGGCAGGAGGGCAATGTGTTCTCCTGCAGCGTGATGCACGA
GGCCCTGCACAATCACTATACCCAGAAGTCCCTGTCCCTGAGCCTGGGCAAG (SEQ
ID NO: 11).

The nucleic acid sequence encoding the amino acid sequence of the heavy chain of Antibody 4-hu4 is set forth in SEQ ID NO: 14:

GAGGTGCAGCTGGTGCAGTCCGGCGCCGAGGTGAAGAAGCCCGGCTCCTCCGTG
AAGGTGAGCTGTAAGGCCAGCGGCGGCACCTTCTCCTCCTTTTCCATCGAGTGGG
TGAGGCAGGCCCCTGGCCAGGGACTGGAGTGGATCGGCGAGATCCTGCCCGGCTC
CAATAGCACCAATTACAACGAGAAGTTTAAGGGCCGGGTGACCTTTACCGCCGAC
GAGTCCACCTCCACCGCCTATATGGAGCTGAGCTCCCTGAGGAGCGAGGACACCG
CTGTGTATTACTGTTCCTCCTACTGGTTTGCTTACTGGGGCCAGGGCACCCTGGTGA
CCGTGTCCTCCGCTTCCACCAAGGGCCCCAGCGTGTTTCCCCTGGCTCCTTGCAGC
CGGAGCACCTCCGAGTCCACCGCTGCTCTGGGCTGCCTGGTGAAGGATTATTTCCC

TGAGCCTGTGACCGTGAGCTGGAACAGCGGCGCTCTGACCTCCGGCGTGCACACC
TTCCCTGCCGTGCTGCAGTCCAGCGGCCTGTATAGCCTGTCCTCCGTGGTGACCGT
GCCCAGCTCCAGCCTGGGCACCAAGACCTATACCTGCAACGTGGATCACAAGCCT
TCCAATACCAAGGTGGACAAGCGGGTGGAGAGCAAGTATGGCCCCCCCTGTCCTC
CTTGCCCTGCTCCTGAGTTCCTGGGCGGCCCCAGCGTCTTCCTGTTTCCCCCTAAG
CCCAAGGACACCCTGATGATCAGCAGGACCCCTGAGGTGACCTGCGTGGTGGTGG
ATGTGTCCCAGGAGGATCCTGAGGTGCAGTTTAACTGGTATGTGGATGGCGTGGAG
GTGCACAACGCCAAGACCAAGCCCCGGGAGGAGCAGTTTAACAGCACCTATAGG
GTGGTGAGCGTGCTGACCGTGCTGCACCAGGATTGGCTGAACGGCAAGGAGTATA
AGTGTAAGGTGAGCAATAAGGGCCTGCCTAGCTCCATCGAGAAGACCATCAGCAA
GGCCAAGGGCCAGCCTAGGGAGCCTCAGGTGTACACCCTGCCTCCCTCCCAGGAG
GAGATGACCAAGAATCAGGTGAGCCTGACCTGTCTGGTGAAGGGCTTTTACCCCT
CCGATATCGCCGTGGAGTGGGAGTCCAATGGCCAGCCTGAGAACAATTATAAGACC
ACCCCCCCCGTGCTGGACTCCGATGGCAGCTTTTTCCTGTACAGCAGGCTGACCGT
GGATAAGAGCCGGTGGCAGGAGGGCAATGTGTTTAGCTGCAGCGTGATGCACGAG
GCCCTGCACAACCACTATACCCAGAAGAGCCTGAGCCTGAGCCTCGGCAAG (SEQ
ID NO: 14).

The amino acid sequence of the heavy chain variable region of Antibody 4-hu5 is set forth in SEQ ID NO: 15:

EVQLVQSGAEVKKPGSSVKVSCKASGGTFSSFSIEWVRQAPGQGLEWIGEILPGSNST
NYNEKFKGRVTFTADTSTNTAYMELSSLRSEDTAVYYCSSYWFAYWGQGTLVTVSS
(SEQ ID NO:15).

The amino acid sequence of the heavy chain of Antibody 4-hu5 is set forth in SEQ ID NO: 16 (IgG4 S228P mutation):

EVQLVQSGAEVKKPGSSVKVSCKASGGTFSSFSIEWVRQAPGQGLEWIGEILPGSNST
NYNEKFKGRVTFTADTSTNTAYMELSSLRSEDTAVYYCSSYWFAYWGQGTLVTVSSA
STKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPS

QEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK (SEQ ID NO: 16)

The nucleic acid sequence encoding the amino acid sequence of the heavy chain of Antibody 4-hu5 is set forth in SEQ ID NO: 17:

GAGGTGCAGCTGGTGCAGTCCGGCGCTGAGGTGAAGAAGCCTGGCTCCTCCGTG
AAGGTGAGCTGCAAGGCTTCCGGCGGCACCTTTTCCAGCTTTAGCATCGAGTGGG
TGAGGCAGGCTCCTGGCCAGGGCCTGGAGTGGATCGGCGAGATCCTGCCTGGCTC
CAACAGCACCAACTACAATGAGAAGTTTAAGGGCCGGGTGACCTTCACCGCTGAC
ACCTCCACCAACACCGCTTATATGGAGCTGAGCTCCCTGCGGAGCGAGGATACCGC
TGTGTACTATTGCAGCAGCTATTGGTTCGCTTACTGGGGCCAGGGCACCCTGGTGA
CCGTGTCCTCCGCCAGCACCAAGGGCCCCTCCGTGTTCCCCCTGGCCCCTTGTAGC
CGGTCCACCTCCGAGAGCACCGCCGCTCTGGGCTGTCTGGTGAAGGATTACTTCC
CCGAGCCTGTGACCGTGTCTTGGAACTCCGGCGCCCTGACCTCCGGCGTGCACAC
ATTCCCTGCTGTGCTGCAGAGCTCCGGCCTGTACTCCCTGAGCAGCGTGGTGACCG
TGCCCTCCTCCAGCCTGGGCACCAAGACCTACACCTGTAATGTGGACCACAAGCC
CTCCAATACCAAGGTGGACAAGAGGGTGGAGTCCAAGTACGGCCCTCCCTGTCCT
CCTTGCCCTGCCCCTGAGTTTCTGGGCGGCCCTAGCGTGTTCCTGTTCCCTCCTAA
GCCTAAGGACACCCTGATGATCAGCCGGACCCCCGAGGTGACCTGTGTGGTGGTG
GATGTGTCCCAGGAGGATCCCGAGGTGCAGTTCAATTGGTACGTGGATGGCGTGG
AGGTGCACAACGCCAAGACCAAGCCTAGGGAGGAGCAGTTTAACTCCACCTACA
GGGTGGTGTCCGTGCTGACCGTGCTGCACCAGGACTGGCTGAATGGCAAGGAGTA
CAAGTGTAAGGTGAGCAACAAGGGCCTGCCTTCCAGCATCGAGAAGACCATCTCC
AAGGCTAAGGGCCAGCCTCGGGAGCCTCAGGTGTACACCCTGCCCCCTAGCCAGG
AGGAGATGACCAAGAACCAGGTGTCCCTGACCTGCCTGGTGAAGGGCTTTTATCC
CTCCGACATCGCCGTGGAGTGGGAGAGCAATGGCCAGCCTGAGAACAATTATAAG
ACCACCCCTCCTGTGCTGGACAGCGATGGCTCCTTCTTTCTGTACTCCCGGCTGAC
CGTGGACAAGAGCAGGTGGCAGGAGGGCAATGTGTTCTCCTGTAGCGTGATGCAC
GAGGCCCTGCACAACCACTACACCCAGAAGTCCCTGTCCCTGTCCCTCGGCAAG(S
EQ ID NO: 17).

The amino acid sequence of the heavy chain variable region of Antibody 4-hu6 is set forth in SEQ ID NO: 18:

EVQLVQSGAEVKKPGSSVKVSCKASGGTFSSFSIEWVRQAPGQGLEWIGEILPGSNST
NYNEKFKGKATFTADTSTNTAYMELSSLRSEDTAVYYCSSYWFAYWGQGTLVTVSS
(SEQ ID NO:18).

The amino acid sequence of the heavy chain of Antibody 4-hu6 is set forth in SEQ ID NO: 19 (IgG4 S228P mutation):

EVQLVQSGAEVKKPGSSVKVSCKASGGTFSSFSIEWVRQAPGQGLEWIGEILPGSNST
NYNEKFKGKATFTADTSTNTAYMELSSLRSEDTAVYYCSSYWFAYWGQGTLVTVSSA
STKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPS
QEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK (SEQ ID NO:19).

The nucleic acid sequence encoding the amino acid sequence of the heavy chain of Antibody 4-hu6 is set forth in SEQ ID NO: 20:

GAGGTGCAGCTGGTGCAGTCCGGCGCCGAGGTGAAGAAGCCTGGCAGCTCCGTG
AAGGTGTCCTGTAAGGCTTCCGGCGGCACCTTCTCCTCCTTTTCCATCGAGTGGGT
GAGGCAGGCCCCCGGCCAGGGATTGGAGTGGATCGGCGAGATCCTGCCTGGCAGC
AACTCCACCAATTATAATGAGAAGTTTAAGGGCAAGGCTACCTTCACCGCTGACAC
CAGCACCAATACCGCCTATATGGAGCTGTCCTCCCTGCGGAGCGAGGACACCGCC
GTGTATTATTGTAGCAGCTATTGGTTCGCCTACTGGGGCCAGGGCACCCTGGTGAC
CGTGAGCAGCGCTAGCACCAAGGGCCCTAGCGTGTTTCCTCTGGCTCCTTGTTCCA
GGTCCACCAGCGAGAGCACCGCTGCCCTGGGCTGCCTGGTGAAGGATTACTTTCC
TGAGCCTGTGACCGTGTCTTGGAACAGCGGCGCTCTGACCAGCGGCGTGCACACC
TTTCCCGCTGTGCTGCAGTCCAGCGGCCTGTATTCCCTGTCCAGCGTGGTGACCGT
GCCCAGCTCCTCCCTGGGCACCAAGACCTATACCTGCAACGTGGATCACAAGCCCT
CCAATACCAAGGTGGACAAGAGGGTGGAGAGCAAGTACGGCCCTCCTTGCCCTCC
CTGTCCTGCTCCTGAGTTCCTGGGCGGCCCTTCCGTGTTTCTGTTTCCCCCTAAGCC
CAAGGATACCCTGATGATCAGCCGGACCCCCGAGGTGACCTGCGTGGTGGTGGAT

GTGTCCCAGGAGGACCCCGAGGTGCAGTTTAATTGGTATGTGGATGGCGTGGAGG
TGCACAACGCTAAGACCAAGCCTAGGGAGGAGCAGTTTAACAGCACCTATCGGGT
GGTGAGCGTGCTGACCGTGCTGCACCAGGACTGGCTGAACGGCAAGGAGTACAA
GTGCAAGGTGTCCAATAAGGGCCTGCCTAGCTCCATCGAGAAGACCATCAGCAAG
GCCAAGGGCCAGCCTAGGGAGCCCCAGGTGTATACCCTGCCCCCTTCCCAGGAGG
AGATGACCAAGAATCAGGTGAGCCTGACCTGTCTGGTGAAGGGCTTTTATCCTAGC
GACATCGCTGTGGAGTGGGAGAGCAATGGCCAGCCTGAGAATAATTACAAGACCA
CCCCCCCTGTGCTGGATTCCGATGGCAGCTTTTTCCTGTATTCCCGGCTGACCGTGG
ATAAGTCCCGGTGGCAGGAGGGCAACGTGTTTAGCTGCTCCGTGATGCACGAGGC
TCTGCACAATCACTACACCCAGAAGTCCCTGAGCCTGTCCCTGGGCAAG (SEQ ID
NO: 20).

The amino acid sequence of the heavy chain variable region of Antibody 4-hu7 is set forth in SEQ ID NO: 21:

EVQLVQSGAEVKKPGSSVKVSCKASGYTFSSFSIEWVRQAPGQGLEWMGEILPGSNS
TNYNEKFKGRVTITADESTSTAYMELSSLRSEDTAVYYCSSYWFAYWGQGTLVTVSS
(SEQ ID NO:21).

The amino acid sequence of the heavy chain of Antibody 4-hu7 is set forth in SEQ ID NO: 22 (IgG4 S228P mutation):

EVQLVQSGAEVKKPGSSVKVSCKASGYTFSSFSIEWVRQAPGQGLEWMGEILPGSNS
TNYNEKFKGRVTITADESTSTAYMELSSLRSEDTAVYYCSSYWFAYWGQGTLVTVSSA
STKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPS
QEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK (SEQ ID NO: 22).

The nucleic acid sequence encoding the amino acid sequence of the heavy chain of Antibody 4-hu7 is set forth in SEQ ID NO: 23:

GAGGTGCAGCTGGTGCAGTCCGGCGCTGAGGTGAAGAAGCCTGGCAGCAGCGTG
AAGGTGAGCTGCAAGGCTTCCGGCTACACCTTTTCCAGCTTCAGCATCGAGTGGG

TGCGGCAGGCTCCCGGCCAGGGATTGGAGTGGATGGGCGAGATCCTGCCTGGCAG
CAACTCCACCAATTACAATGAGAAGTTCAAGGGCAGGGTGACCATCACCGCTGAC
GAGAGCACCTCCACCGCTTACATGGAGCTGAGCAGCCTGAGGTCCGAGGATACCG
CCGTGTATTACTGCAGCAGCTATTGGTTTGCCTACTGGGGCCAGGGCACCCTGGTG
ACCGTGAGCTCCGCCAGCACCAAGGGCCCCAGCGTGTTTCCCCTGGCTCCTTGCA
GCAGGTCCACCAGCGAGAGCACCGCCGCTCTGGGCTGTCTGGTGAAGGACTACTT
CCCCGAGCCTGTGACCGTGTCTTGGAATAGCGGCGCTCTGACCAGCGGCGTGCAC
ACCTTTCCTGCTGTGCTGCAGTCCTCCGGCCTGTACTCCCTGTCCAGCGTGGTGAC
CGTGCCCTCCTCCTCCCTGGGCACCAAGACCTATACCTGCAATGTGGATCACAAGC
CTAGCAATACCAAGGTGGACAAGAGGGTGGAGAGCAAGTACGGCCCCCCTTGCCC
TCCTTGCCCCGCTCCTGAGTTCCTGGGCGGCCCTTCCGTGTTCCTGTTTCCCCCTAA
GCCCAAGGACACCCTGATGATCTCCCGGACCCCCGAGGTGACCTGTGTGGTGGTG
GATGTGAGCCAGGAGGACCCTGAGGTGCAGTTCAATTGGTACGTGGATGGCGTGG
AGGTGCACAACGCCAAGACCAAGCCTAGGGAGGAGCAGTTCAACAGCACCTACC
GGGTGGTGTCCGTGCTGACCGTGCTGCACCAGGACTGGCTGAATGGCAAGGAGTA
CAAGTGCAAGGTGTCCAATAAGGGCCTGCCTTCCTCCATCGAGAAGACCATCAGC
AAGGCCAAGGGCCAGCCTCGGGAGCCTCAGGTGTATACCCTGCCTCCCAGCCAGG
AGGAGATGACCAAGAACCAGGTGTCCCTGACCTGCCTGGTGAAGGGCTTTTACCC
TTCCGATATCGCCGTGGAGTGGGAGAGCAATGGCCAGCCTGAGAATAATTACAAG
ACCACCCCTCCTGTGCTGGATAGCGACGGCAGCTTTTTCCTGTACAGCCGGCTGAC
CGTGGATAAGAGCCGGTGGCAGGAGGGCAACGTGTTCAGCTGCAGCGTGATGCAC
GAGGCTCTGCACAACCACTATACCCAGAAGAGCCTGTCCCTGAGCCTGGGCAAG
(SEQ ID NO: 23).

The amino acid sequence of the heavy chain variable region of Antibody 4-hu8 is set forth in SEQ ID NO: 24:

EVQLVQSGAEVKKPGSSVKVSCKASGYTFSSFSIEWVRQAPGQGLEWMGEILPGSNS
TNYNEKFKGRVTFTADESTSTAYMELSSLRSEDTAVYYCSSYWFAYWGQGTLVTVSS
(SEQ ID NO: 24).

The amino acid sequence of the heavy chain of Antibody 4-hu8 is set forth in SEQ ID NO: 25 (IgG4 S228P mutation):

EVQLVQSGAEVKKPGSSVKVSCKASGYTFSSFSIEWVRQAPGQGLEWMGEILPGSNS
TNYNEKFKGRVTFTADESTSTAYMELSSLRSEDTAVYYCSSYWFAYWGQGTLVTVSS
ASTKGPSVFPLAPCSRSTSESTAALGCLVKDYFPEPVTVSWNSGALTSGVHTFPAVLQS
SGLYSLSSVVTVPSSSLGTKTYTCNVDHKPSNTKVDKRVESKYGPPCPPCPAPEFLGGP
SVFLFPPKPKDTLMISRTPEVTCVVVDVSQEDPEVQFNWYVDGVEVHNAKTKPREEQ
FNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLT
VDKSRWQEGNVFSCSVMHEALHNHYTQKSLSLSLGK (SEQ ID NO: 25).

The nucleic acid sequence encoding the amino acid sequence of the heavy chain of Antibody 4-hu8 is set forth in SEQ ID NO: 26:

GAGGTGCAGCTGGTGCAGAGCGGCGCTGAGGTGAAGAAGCCCGGCTCCAGCGTG
AAGGTGTCCTGCAAGGCTTCCGGCTATACCTTCTCCAGCTTTAGCATCGAGTGGGT
GAGGCAGGCTCCTGGCCAGGGCCTGGAGTGGATGGGCGAGATCCTGCCTGGCAGC
AATTCCACCAACTATAACGAGAAGTTCAAGGGCCGGGTGACCTTCACCGCCGATG
AGTCCACCTCCACCGCCTACATGGAGCTGAGCTCCCTGCGGTCCGAGGATACCGCC
GTGTACTATTGCAGCAGCTATTGGTTCGCTTATTGGGGCCAGGGCACCCTGGTGAC
CGTGAGCTCCGCTTCCACCAAGGGCCCTTCCGTGTTTCCCCTGGCCCCCTGCTCCC
GGTCCACCTCCGAATCCACCGCCGCCCTGGGCTGCCTGGTGAAGGACTACTTCCCT
GAGCCCGTGACCGTGTCCTGGAACTCCGGCGCCCTGACCAGCGGCGTGCACACAT
TTCCCGCTGTGCTGCAGTCCTCCGGCCTGTATAGCCTGTCCAGCGTGGTGACCGTG
CCTAGCTCCAGCCTGGGCACCAAGACCTACACCTGTAATGTGGATCACAAGCCCA
GCAATACCAAGGTGGATAAGAGGGTGGAGTCCAAGTACGGCCCCCCTTGTCCTCC
TTGTCCCGCCCCAGAGTTCCTGGGCGGCCCATCTGTGTTTCTGTTTCCCCCCAAGC
CTAAGGACACCCTGATGATCTCCCGGACCCCTGAGGTGACCTGTGTGGTGGTGGA
CGTGTCCCAGGAGGATCCTGAGGTGCAGTTCAATTGGTATGTGGACGGCGTGGAG
GTGCACAACGCCAAGACCAAGCCCAGGGAGGAGCAGTTTAACAGCACCTATCGG
GTGGTGTCCGTGCTGACCGTGCTGCACCAGGACTGGCTGAATGGCAAGGAGTATA
AGTGCAAGGTGAGCAATAAGGGCCTGCCCTCCAGCATCGAGAAGACCATCTCCAA
GGCTAAGGGCCAGCCCCGGGAGCCTCAGGTGTACACCCTGCCTCCCAGCCAGGAG
GAGATGACCAAGAACCAGGTGTCCCTGACCTGTCTGGTGAAGGGCTTTTATCCCA

GCGACATCGCCGTGGAGTGGGAGTCCAACGGCCAGCCTGAGAACAACTACAAGA
CCACCCCTCCCGTGCTGGACTCCGATGGCTCCTTTTTCCTGTATTCCAGGCTGACC
GTGGATAAGAGCAGGTGGCAGGAGGGCAACGTGTTTAGCTGCTCCGTGATGCACG
AGGCCCTGCACAATCACTATACCCAGAAGTCCCTGAGCCTGAGCCTGGGCAAG
(SEQ ID NO: 26).

The amino acid sequence of the heavy chain variable region of Antibody 4-hu9 is set forth in SEQ ID NO: 27:

EVQLVQSGAEVKKPGSSVKVSCKASGYTFSSFSIEWVRQAPGQGLEWIGEILPGSNST
NYNEKFKGRVTFTADESTSTAYMELSSLRSEDTAVYYCSSYWFAYWGQGTLVTVSS
(SEQ ID NO: 50).

The amino acid sequence of the heavy chain of Antibody 4-hu9 is set forth in SEQ ID NO: 28 (IgG4 S228P mutation):

EVQLVQSGAEVKKPGSSVKVSCKASGYTFSSFSIEWVRQAPGQGLEWIGEILPGSNST
NYNEKFKGRVTFTADESTSTAYMELSSLRSEDTAVYYCSSYWFAYWGQGTLVTVSSA
STKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPS
QEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK (SEQ ID NO: 28).

The nucleic acid sequence encoding the amino acid sequence of the heavy chain of Antibody 4-hu9 is set forth in SEQ ID NO: 29:

GAGGTGCAGCTGGTGCAGAGCGGCGCCGAGGTGAAGAAGCCCGGCTCCAGCGTG
AAGGTGTCCTGCAAGGCTTCCGGCTATACCTTCTCCAGCTTCAGCATCGAGTGGGT
GAGGCAGGCCCCCGGCCAGGGATTGGAGTGGATCGGCGAGATCCTGCCTGGCAGC
AATAGCACCAACTATAATGAGAAGTTCAAGGGCAGGGTGACCTTCACCGCCGATG
AGAGCACCTCCACCGCTTACATGGAGCTGAGCAGCCTGAGGAGCGAGGATACCGC
TGTGTATTATTGTAGCAGCTATTGGTTTGCCTACTGGGGCCAGGGCACCCTGGTGAC
CGTGTCCAGCGCCAGCACCAAGGGCCCCAGCGTGTTCCCTCTGGCCCCTTGTTCC
CGGTCCACCTCCGAGAGCACCGCCGCTCTGGGCTGCCTGGTGAAGGACTATTTCC

CTGAGCCCGTGACCGTGAGCTGGAACTCCGGCGCTCTGACCAGCGGCGTGCACAC
CTTCCCTGCCGTGCTGCAGTCCAGCGGCCTGTACTCCCTGTCCAGCGTGGTGACCG
TGCCCAGCTCCAGCCTGGGCACCAAGACCTACACCTGCAACGTGGATCACAAGCC
CTCCAATACCAAGGTGGACAAGAGGGTGGAGAGCAAGTATGGCCCTCCCTGTCCC
CCCTGCCCTGCCCCTGAATTTCTGGGCGGCCCCTCCGTGTTTCTGTTTCCTCCTAAG
CCTAAGGATACCCTGATGATCTCCCGGACCCCTGAGGTGACCTGTGTGGTGGTGGA
TGTGAGCCAGGAGGACCCCGAGGTGCAGTTTAACTGGTACGTGGACGGCGTGGA
GGTGCACAATGCTAAGACCAAGCCCCGGGAGGAGCAGTTCAATAGCACCTATAGG
GTGGTGAGCGTGCTGACCGTGCTGCACCAGGACTGGCTGAACGGCAAGGAGTATA
AGTGCAAGGTGAGCAATAAGGGCCTGCCCTCCTCCATCGAGAAGACCATCTCCAA
GGCTAAGGGCCAGCCCCGGGAGCCTCAGGTGTACACCCTGCCCCCTAGCCAGGAG
GAGATGACCAAGAATCAGGTGTCCCTGACCTGCCTGGTGAAAGGCTTTTACCCCT
CCGATATCGCCGTGGAGTGGGAGTCCAATGGCCAGCCTGAGAACAATTACAAGAC
CACCCCTCCCGTGCTGGATTCCGACGGCAGCTTCTTTCTGTATTCCAGGCTGACCG
TGGATAAGTCCCGGTGGCAGGAGGGCAACGTGTTTAGCTGCAGCGTGATGCACGA
GGCCCTGCACAATCACTACACCCAGAAGTCCCTGAGCCTGTCCCTGGGCAAG
(SEQ ID NO: 29).

The amino acid sequence of the heavy chain variable region of Antibody 4-hu10 is set forth in SEQ ID NO: 30:

EVQLVQSGAEVKKPGSSVKVSCKASGYTFSSFSIEWVRQAPGQGLEWIGEILPGSNST
NYNEKFKGRVTFTADTSTNTAYMELSSLRSEDTAVYYCSSYWFAYWGQGTLVTVSS
(SEQ ID NO: 30).

The amino acid sequence of the heavy chain of Antibody 4-hu10 is set forth in SEQ ID NO: 31 (IgG4 S228P mutation):

EVQLVQSGAEVKKPGSSVKVSCKASGYTFSSFSIEWVRQAPGQGLEWIGEILPGSNST
NYNEKFKGRVTFTADTSTNTAYMELSSLRSEDTAVYYCSSYWFAYWGQGTLVTVSSA
STKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPS

QEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK (SEQ ID NO: 31).

The nucleic acid sequence encoding the amino acid sequence of the heavy chain of Antibody 4-hu10 is set forth in SEQ ID NO: 32:

GAGGTGCAGCTGGTGCAGAGCGGCGCTGAGGTGAAGAAGCCTGGCAGCAGCGTG
AAGGTGAGCTGCAAGGCTTCCGGCTACACCTTTTCCAGCTTCAGCATCGAGTGGG
TGAGGCAGGCCCCTGGCCAGGGACTGGAGTGGATCGGCGAGATCCTGCCTGGCAG
CAATAGCACCAATTACAACGAGAAGTTCAAGGGCCGGGTGACCTTCACCGCCGAC
ACCAGCACCAATACCGCCTATATGGAGCTGAGCTCCCTGAGGTCCGAGGACACCG
CTGTGTATTATTGCTCCAGCTATTGGTTCGCCTACTGGGGCCAGGGCACCCTGGTGA
CCGTGTCCTCCGCCAGCACCAAGGGCCCCAGCGTGTTTCCTCTGGCCCCTTGCAG
CAGGTCCACCAGCGAGAGCACCGCCGCTCTGGGCTGCCTGGTGAAGGATTACTTC
CCTGAGCCTGTGACCGTGTCTTGGAACAGCGGCGCCCTGACCTCCGGCGTGCACA
CATTTCCTGCTGTGCTGCAGAGCAGCGGCCTGTATAGCCTGAGCAGCGTGGTGACC
GTGCCTAGCAGCAGCCTGGGCACCAAGACCTACACCTGCAATGTGGATCACAAGC
CCTCCAACACCAAGGTGGACAAGCGGGTGGAGAGCAAGTACGGCCCTCCCTGCC
CCCCCTGTCCTGCTCCTGAGTTTCTGGGCGGCCCTAGCGTGTTCCTGTTCCCTCCTA
AGCCCAAGGACACCCTGATGATCAGCAGGACCCCTGAGGTGACCTGCGTGGTGGT
GGACGTGTCCCAGGAGGATCCCGAGGTGCAGTTCAACTGGTACGTGGACGGCGTG
GAGGTGCACAATGCCAAGACCAAGCCTAGGGAGGAGCAGTTTAACAGCACCTATA
GGGTGGTGAGCGTGCTGACCGTGCTGCACCAGGACTGGCTGAACGGCAAGGAGT
ATAAGTGTAAGGTGTCCAACAAGGGCCTGCCTTCCTCCATCGAGAAGACCATCTCC
AAGGCTAAGGGCCAGCCTAGGGAGCCTCAGGTGTATACCCTGCCTCCCTCCCAGG
AGGAGATGACCAAGAATCAGGTGTCCCTGACCTGCCTGGTGAAAGGCTTTTACCC
CAGCGACATCGCTGTGGAGTGGGAGAGCAACGGCCAGCCCGAGAATAATTACAAG
ACCACCCCCCCTGTGCTGGATTCCGACGGCAGCTTCTTTCTGTACTCCAGGCTGAC
CGTGGACAAGTCCAGGTGGCAGGAGGGCAACGTGTTCAGCTGCAGCGTGATGCA
CGAGGCTCTGCACAACCACTACACCCAGAAGTCCCTGTCCCTGAGCCTCGGCAAG
(SEQ ID NO: 32).

The amino acid sequence of the heavy chain variable region of Antibody 4-hu11 is set forth in SEQ ID NO: 33:

EVQLVQSGAEVKKPGSSVKVSCKASGYTFSSFSIEWVRQAPGQGLEWIGEILPGSNST
NYNEKFKGKATFTADTSTNTAYMELSSLRSEDTAVYYCSSYWFAYWGQGTLVTVSS
(SEQ ID NO: 33).

The amino acid sequence of the heavy chain of Antibody 4-hu11 is set forth in SEQ ID NO: 34 (IgG4 S228P mutation):

EVQLVQSGAEVKKPGSSVKVSCKASGYTFSSFSIEWVRQAPGQGLEWIGEILPGSNST
NYNEKFKGKATFTADTSTNTAYMELSSLRSEDTAVYYCSSYWFAYWGQGTLVTVSSA
STKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPS
QEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK (SEQ ID NO: 34).

The nucleic acid sequence encoding the amino acid sequence of the heavy chain of Antibody 4-hu11 is set forth in SEQ ID NO: 35:

GAGGTGCAGCTGGTGCAGTCCGGCGCCGAGGTGAAGAAGCCCGGCAGCAGCGTG
AAGGTGAGCTGCAAGGCTTCCGGCTATACCTTCAGCTCCTTCAGCATCGAGTGGGT
GCGGCAGGCTCCTGGCCAGGGACTGGAGTGGATCGGCGAGATCCTGCCCGGCAGC
AATTCCACCAACTATAATGAGAAGTTCAAGGGCAAGGCTACCTTCACCGCCGACA
CCTCCACCAATACCGCCTATATGGAGCTGTCCAGCCTGAGGAGCGAGGACACCGCT
GTGTACTACTGCTCCAGCTACTGGTTTGCCTATTGGGGCCAGGGCACCCTGGTGAC
CGTGAGCAGCGCTTCCACCAAGGGCCCCTCCGTGTTCCCTCTGGCCCCCTGTAGC
AGGAGCACCTCCGAGTCCACCGCTGCCCTGGGCTGCCTGGTGAAGGACTATTTCC
CTGAGCCTGTGACCGTGTCCTGGAACAGCGGCGCTCTGACCTCCGGCGTGCACAC
CTTCCCCGCTGTGCTGCAGTCCTCCGGCCTGTACAGCCTGAGCTCCGTGGTGACCG
TGCCTAGCAGCAGCCTGGGCACCAAGACCTATACCTGCAACGTGGATCACAAGCC
TAGCAACACCAAGGTGGACAAGCGGGTGGAGTCCAAGTACGGCCCTCCCTGTCCC
CCTTGCCCCGCTCCTGAGTTTCTGGGCGGCCCTTCCGTGTTTCTGTTCCCCCCTAA
GCCCAAGGATACCCTGATGATCAGCCGGACCCCCGAGGTGACCTGCGTGGTGGTG

GATGTGTCCCAGGAGGACCCCGAGGTGCAGTTCAACTGGTACGTGGACGGCGTGG
AGGTGCACAATGCCAAGACCAAGCCTCGGGAGGAGCAGTTTAACAGCACCTATCG
GGTGGTGAGCGTGCTGACCGTGCTGCACCAGGATTGGCTGAACGGCAAGGAGTAT
AAGTGCAAGGTGTCCAATAAGGGCCTGCCTAGCAGCATCGAGAAGACCATCAGCA
AGGCTAAGGGCCAGCCCCGGGAGCCCCAGGTTTACACCCTGCCCCCTAGCCAGGA
GGAGATGACCAAGAATCAGGTGTCCCTGACCTGCCTGGTGAAAGGCTTCTATCCCT
CCGACATCGCTGTGGAGTGGGAGTCCAATGGCCAGCCCGAGAACAACTACAAGAC
CACCCCTCCCGTGCTGGATAGCGACGGCAGCTTCTTTCTGTACTCCAGGCTGACCG
TGGACAAGAGCCGGTGGCAGGAGGGCAATGTGTTCTCCTGTTCCGTGATGCACGA
GGCCCTGCACAATCACTACACCCAGAAGTCCCTGTCCCTGAGCCTCGGCAAG (SEQ
ID NO: 35).

The amino acid sequence of the light chain variable region of the humanized Antibody 4 is set forth in SEQ ID NO: 36:

DIVMTQSPDSLAVSLGERATINCKSSQSLLNSGNQKNYLAWYQQKPGQPPKLLIYGAS
TRESGVPDRFSGSGSGTDFTLTISSLQAEDVAVYYCQNAHSYPPTFGQGTKLEIK (SEQ
ID NO: 36).

The amino acid sequence of the light chain of the humanized Antibody 4 is set forth in SEQ ID NO: 37:

DIVMTQSPDSLAVSLGERATINCKSSQSLLNSGNQKNYLAWYQQKPGQPPKLLIYGAS
TRESGVPDRFSGSGSGTDFTLTISSLQAEDVAVYYCQNAHSYPPTFGQGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC (SEQ ID NO: 37).

The nucleic acid sequence encoding the amino acid sequence of the light chain of the humanized Antibody 4 is set forth in SEQ ID NO: 38:

GACATCGTGATGACCCAGAGCCCCGATTCCCTGGCCGTGTCCCTGGGCGAGAGGG
CTACCATCAATTGCAAGTCCTCCCAGAGCCTGCTGAATAGCGGCAACCAGAAGAA
CTACCTGGCCTGGTACCAGCAGAAGCCCGGCCAGCCTCCTAAGCTGCTGATCTACG
GCGCCTCCACCCGGGAGAGCGGCGTTCCTGACAGGTTTAGCGGCTCCGGCAGCGG
CACCGATTTCACCCTGACCATCTCCAGCCTGCAGGCCGAGGACGTGGCCGTGTATT
ATTGCCAGAATGCCCACTCCTATCCCCCCACCTTTGGCCAGGGCACCAAGCTGGAG

ATCAAGCGGACCGTGGCCGCCCCTTCCGTGTTTATCTTCCCCCCCTCCGACGAGCA
GCTGAAGTCCGGCACCGCCAGCGTGGTGTGTCTGCTGAACAACTTCTACCCCAGG
GAGGCTAAGGTGCAGTGGAAGGTGGATAACGCCCTGCAGTCCGGCAACAGCCAG
GAGAGCGTGACCGAGCAGGACTCCAAGGACTCCACCTACTCCCTGTCCTCCACCC
TGACCCTGTCCAAGGCCGATTATGAGAAGCACAAGGTGTACGCCTGCGAGGTGAC
CCACCAGGGCCTGAGCTCCCCCGTGACCAAGTCCTTCAATAGGGGCGAGTGC
(SEQ ID NO: 38).

The antibody or antibody fragment mentioned above is isolatable. The term "isolatable" means a condition that is substantially free of antibodies or antibody fragments having different antigen specificities other than the antibody provided. Moreover, an isolatable antibody or antibody fragment can be substantially free of other cellular material and/or chemicals. Thus, in some aspects, the provided antibody is an isolated antibody that has been isolated from the antibodies having different specificities. The isolated antibody may be a monoclonal antibody. The isolated antibody may be a recombinant monoclonal antibody. However, an isolated antibody that specifically binds to a target epitope, isotype, or variant may be cross-reactive with other relevant antigens, e.g., derived from other species (e.g., species homologs).

As used herein, the at least one amino acid substitution may be one amino acid substitution, two amino acid substitutions, three amino acid substitutions, four amino acid substitutions, or five amino acid substitutions, or even more. According to a preferred embodiment of the present disclosure, the amino acid substitution is preferably a conservative amino acid substitution. The conservative amino acid substitution is an amino acid substitution that does not impair the binding of the antibody to the PVRIG antigen.

Conservative amino acid substitutions include the substitution of an amino acid in one class with an amino acid in the same class, where the class is defined by common amino acid side chain physicochemical properties and high substitution frequencies in homologous proteins found in nature, as determined, for example, by standard Dayhoff frequency exchange matrices or BLOSUM matrices. Amino acid side chains have been classified into six major classes including: class I (Cys); class II (Ser, Thr, Pro, Ala, Gly); class III (Asn, Asp, Gln, Glu); class IV (His, Arg, Lys); class V (Ile, Leu, Val, Met), and Class VI (Phe, Tyr, Trp). For example, substitution of Asp with another class III residue such as Asn, Gln, or Glu is a conservative substitution. Thus, a predicted nonessential amino acid residue in an anti-EGFR antibody is preferably substituted with another amino acid residue from the same class. Methods for identifying conservative substitutions of nucleotides and amino acids that do not eliminate antigen binding are well known in the art.

The present disclosure also provides an isolatable nucleic acid encoding the antibody or antibody fragment as described above. The mentioned isolatable nucleic acid means that the nucleic acid can be substantially isolated from other materials and is substantially free of materials or chemicals. The nucleic acid may be present in intact cells, in cell lysates, or in partially purified or substantially pure form. A nucleic acid is "isolated" or "substantially pure" when purified from other cellular components or other impurities, such as other cellular nucleic acids or proteins, by standard techniques including alkaline/SDS treatment, CsCl banding, column chromatography, agarose gel electrophoresis, and other techniques well known in the art.

The present disclosure also provides an expression vector including the nucleic acid. A wide variety of expression vectors can be used to express polynucleotides encoding the antibody or binding fragment mentioned herein. Viral-based and non-viral expression vectors can be used to produce antibodies in mammalian host cells. Non-viral vectors and systems include plasmids, episomal vectors, usually with expression cassettes for expression of proteins or RNA, and artificial human chromosomes (see, e.g., Harrington et al., (1997) Nat Genet 15:345). For example, non-viral vectors useful for expression of polynucleotides and polypeptides in mammalian (e.g. human) cells include pThioHis A,B&C, pcDNA3.1/His, pEBVHis A,B&C (Invitrogen,San Diego,CA), MPSV vectors, and a number of other vectors known in the art for expression of other proteins. Useful viral vectors include retrovirus, adenovirus, adeno-associated virus, and herpes virus based vectors, and SV40, papillomavirus, HBP Epstein Barr virus, cowpox virus, and semliki forest virus (SFV) based vectors. See Brent et.al,(1995)Annu.Rev.Microbiol.49:807; and Rosenfeld et.al,(1992)Cell 68:143.

The expression vector contains a promoter operably linked to a polynucleotide encoding the antibody or antibody fragment and other regulatory sequences (e.g., enhancers). In some embodiments, inducible promoters may be used to prevent expression of inserted sequences beyond the inducing conditions. Inducible promoters include, but are not limited to, arabinose, lacZ, a metallothionein promoter, or a heat shock promoter. In addition to the promoter, additional regulatory elements may be required or needed for efficient expression of the antibody or antibody fragment. These elements typically include a ATG start codon and an adjacent ribosome binding site or other sequence. In addition, expression in mammalian host cells may be increased by the inclusion of enhancers which are appropriate for the cell system employed, such as the SV40 enhancer or the CMV enhancer.

The present disclosure also provides a host cell expressing the antibody or antibody fragment as described above. According to an embodiment of the present disclosure, the host cell contains the expression vector as described above, and can be used to express the antibody mentioned in the present disclosure. The expression vector may be integrated into the genome of the host cell. The host cell may be a mammalian cell, including but not limited to CHO or HEK293 cells.

The host cell for carrying and expressing the antibody or antibody fragment can be prokaryocyte or eukaryocyte. For example, the prokaryotic host cell is E. coli. Other suitable microbial hosts include Bacillus, such as Bacillus subtilis, and other Enterobacteriaceae, such as Salmonella, Serratia, and various Pseudomonas species. In these prokaryotic hosts, expression vectors may also be produced, which typically contain expression control sequences compatible with the host cell. In addition, these host cells also contain promoters, such as the lactose promoter system, the tryptophan (trp) promoter system, the β-lactamase promoter system, or the promoter system derived from bacteriophage λ. Typically, promoters optionally cooperate with operator sequences to control expression, and have a ribosome binding site sequence or the like for initiating and completing transcription and translation. Other microorganisms such as yeast can also be used to express the polypeptides of the present disclosure. Insect cells may also be used in conjunction with baculovirus vectors.

In some preferred embodiments, mammalian host cells are used to express and produce the anti-PVRIG antibody or antibody fragment of the present disclosure. For example, they may be hybridoma cell lines expressing endogenous immunoglobulin genes. Preferably, mammalian cell lines carrying exogenous expression vectors are used, including any normal lethal or normal or abnormal immortalized animal or human cells, e.g., SP2/0 myeloma cells, CHO cells, HeLa cells, PER. C6 cells, COS cells, HKB11 cells, NS0 cells. For example, a variety of suitable host cell lines capable of secreting intact immunoglobulins have been developed, including CHO cell lines, various Cos cell lines, HeLa cells, myeloma cell lines, transformed B-cells and hybridomas. Suitable promoters contained in host cells may be constitutive, cell type specific, stage specific and/or regulatable or adjustable. Useful promoters include, but are not limited to, the metallothionein promoter, the constitutive adenovirus major late promoter, the dexamethasone inducible MMTV promoter, the SV40 promoter, the MRP pollll promoter, the constitutive MPSV promoter, the constitutive CMV promoter, and promoter-enhancer combinations known in the art.

A vector containing a polynucleotide sequence of interest (e.g., a nucleic acid encoding an antibody polypeptide and expression control sequences) can be transformed into a host cell by well-known methods, depending on the type of the cellular host. For example, calcium chloride transfection is often used for prokaryotic cells, while calcium phosphate treatment or electroporation may be used for other cellular hosts. Other methods may be, for example, electroporation, calcium phosphate treatment, liposome-mediated transformation, injection and microinjection, virosomes, immunoliposomes, polycationic nucleic acid conjugates, naked DNA, artificial virosomes, etc.

The present disclosure also provides a pharmaceutical composition including the antibody as described above and a pharmaceutically acceptable carrier.

The pharmaceutically acceptable carrier included any and all physiologically compatible solvents, dispersion media, coatings, antibacterial and antifungal agents, isotonizing and absorption delaying agents, and the like. Preferably, the carrier is suitable for intravenous, intramuscular, subcutaneous, parenteral, spinal or epidermal administration (e.g., administration by injection or infusion). Depending on the route of administration, the active compound, i.e., the antibody, may be encapsulated in a material to protect the compound from the action of acids and other natural conditions that may inactivate the compound.

The provided pharmaceutical composition can be administered by a variety of methods known in the art. The antibody can be prepared with suitable carriers, such as controlled release formulations, including implants, transdermal patches, and microencapsulated delivery systems. Biodegradable and biocompatible polymers can be used, such as ethylene vinyl acetate, polyanhydrides, polyglycolic acid, collagen, poly(ortho esters), and polylactic acid. Many methods for preparing such formulations are patented or generally known to those skilled in the art.

According to embodiments of the present disclosure, the provided antibody may be coated with or co-administered with a material to prevent inactivation thereof. For example, the composition can be administered to a subject in an appropriate carrier, such as a liposome or diluent. The acceptable diluent includes saline and an aqueous buffer. The liposome includes the water-in-oil-in-water CGF emulsion and conventional liposomes.

According to embodiments of the present disclosure, the pharmaceutically acceptable carrier includes a sterile aqueous solution or dispersion and the like prepared as a sterile injectable solution or sterile powder. The pharmaceutical composition provided herein can be formulated into a pharmaceutically acceptable dosage form by a conventional method known to those skilled in the art.

The present disclosure also provides use of the antibody as described above or the pharmaceutical composition as described above in the preparation of a medicament for the treatment of cancer.

The present disclosure also provides the antibody or antibody fragment as described above or the pharmaceutical composition as described above for use in preventing or treating cancer, including administering to a subject an effective amount of the antibody as described above or the pharmaceutical composition as described above.

"Therapeutically effective amount" or "effective amount" refers to the amount of PVRIG antibody required to elicit the desired biological response. According to the present disclosure, the therapeutically effective amount refers to the amount of PVRIG antibody required to treat and/or prevent a disease.

The therapeutically effective amount of the antibody provided herein depends on the relative activities of the antibody and the combination (e.g., the activity in inhibiting cell growth), varies depending on the subject and disease condition being treated, the weight and age of the subject, the severity of the disease condition, the manner of administration, and the like, and can be readily determined by one of ordinary skill in the art. Dosages for administration may be in the following ranges: for example, from about 1 ng to about 6000 mg, from about 5 ng to about 5500 mg, from about 10 ng to about 5000 mg, from about 20 ng to about 4500 mg, from about 30 ng to about 5000 mg, from about 300 ng to about 4,500 mg, from about 500 ng to about 4,000 mg, from about 1 µg to about 3,500 mg, from about 5 µg to about 3,000 mg, from about 10 µg to about 2,600 mg, from about 20 µg to about 2,575 mg, from about 30 µg to about 2,550 mg, from about 40 µg to about 2,500 mg, from about 50 µg to about 2,475 mg, from about 100 µg to about 2,450 mg, from about 200 µg to about 2,425 mg, from about 300 µg to about 2,000, from about 400 µg to about 1,175 mg, from about 500 µg to about 1,150 mg, from about 0.5 mg to about 1,125 mg, from about 1 mg to about 1,100 mg, from about 1.25 mg to about 1,075 mg, from about 1.5 mg to about 1,050 mg, from about 2.0 mg to about 1,025 mg, from about 2.5 mg to about 1,000 mg, from about 3.0 mg to about 975 mg, from about 3.5 mg to about 950 mg, from about 4.0 mg to about 925 mg, from about 4.5 mg to about 900 mg, from about 5 mg to about 875 mg, from about 10 mg to about 850 mg, from about 20 mg to about 825 mg, from about 30 mg to about 800 mg, from about 40 mg to about 775 mg, from about 50 mg to about 750 mg, from about 100 mg to about 725 mg, from about 200 mg to about 700 mg, from about 300 mg to about 675 mg, from about 400 mg to about 650 mg, from about 500 mg or about 525 mg to about 625 mg of the antibody.

As used herein, "subject" refers to any animal, including rodents, such as mice or rats, primates, such as Macaca fascicularis, Macaca mulatta, or Homo sapiens. Preferably, the subject is a primate, more preferably a human being. As used herein, "preventing" refers to a method directed at preventing or delaying the onset of a disease.

As used herein, examples of cancer include, but are not limited to, carcinoma, lymphoma, blastoma, sarcoma, and leukemia. More specific examples include: squamous cell cancer, small-cell lung cancer, non-small cell lung cancer, gastric cancer, pancreatic cancer, glial cell tumors such as glioblastoma and neurofibromatosis, cervical cancer, ovarian cancer, liver cancer, bladder cancer, hepatoma, breast cancer, colon cancer, melanoma, colorectal cancer, endometrial cancer, salivary gland cancer, kidney cancer, renal carcinoma, prostate cancer, vulval cancer, thyroid cancer, liver cancer, as well as various types of head and neck cancer. In particular embodiments, the cancer treated or diagnosed using the methods disclosed herein is selected from melanoma, breast cancer, ovarian cancer, renal carcinoma, gastrointestinal/colon cancer, lung cancer, and prostate cancer. According to a preferred embodiment of the present disclosure, the cancer includes but is not limited to melanoma, lung cancer, head and neck cancer, colorectal cancer, pancreatic cancer, gastric cancer, renal carcinoma, bladder cancer, prostate cancer, breast cancer, ovarian cancer, or liver cancer.

The present disclosure also provides a pharmaceutical combination which may contain other drugs for the treatment of cancer, in addition to the antibody or antibody fragment mentioned above. The composition can be administered alone or in combination therapy, i.e., in combination with other agents. For example, the combination therapy can include a composition provided herein and at least one or more additional therapeutic agents such as anti-cancer agents described in the present disclosure. The composition may be administered in conjunction with radiation therapy and/or surgery. The particular combination of anti-EGFR antibodies may also be administered separately or sequentially, with or without additional therapeutic agents.

For example, the additional drugs provided to treat cancer can be antibodies, such as an anti-CTLA-4 antibody, an anti-PD-Ll antibody, an anti-LAG-3 antibody, an anti-TIM-3 antibody, an anti-BTLA antibody, or other anti-tumor antibody combinations.

Exemplary antibodies provided herein, including murine or humanized antibodies, are capable of binding to human PVRIG protein with high affinity and promoting tumor cell killing by human PBMC. In addition, the provided antibodies also exhibit tumor growth inhibitory activity in vivo.

It will be understood by those skilled in the art that the following examples are merely illustrative of the present disclosure and are not to be construed as limiting the scope of the present disclosure. Examples, where specific techniques or conditions are not specified, are implemented in accordance with techniques or conditions described in the literature in the art or according to the product instructions. All of the used agents or instruments of which the manufacturers are not specified are conventional commercially-available products.

### Example 1

### (I) Preparation of monoclonal antibodies against human PVRIG

The monoclonal antibodies against PVRIG were prepared by the conventional hybridoma cell fusion technology that was slightly adjusted, as follows:

### (1) Preparation of recombinant fusion proteins

A full-length gene encoding the human PVRIG protein (as set forth in SEQ ID NO: 45) was artificially synthesized, the protein fragment set forth in SEQ ID NO: 46 was amplified using PCR, a protein fragment of a human IgG1 constant region (as set forth in SEQ ID NO: 47) was introduced at the C-terminus of the sequence of interest and cloned into a pLVX-IRES-zsGreen vector, the HEK293T cell line was infected by packaging lentivirus to obtain overexpressed human PVRIG-human IgG1 Fc fusion protein, and then the cell culture supernatant was harvested after conventional cell culture, and purified by a Protein G affinity chromatography method (GE Healthcare) to obtain a recombinant human PVRIG-human IgG1 Fc fusion protein with a purity of greater than 90%. Mass spectrometry identification showed that the peptide fragment of interest protein matched the sequence of PVRIG, which proved that the recombinant protein was a recombinant human PVRIG-human IgG1 Fc fusion protein.

Human PVRIG full length amino acid sequence:

MRTEAQVPALQPPEPGLEGAMGHRTLVLPWVLLTLCVTAGTPEVWVQVRMEATELSS
FTIRCGFLGSGSISLVTVSWGGPNGAGGTTLAVLHPERGIRQWAPARQARWETQSSISL
ILEGSGASSPCANTTFCCKFASFPEGSWEACGSLPPSSDPGLSAPPTPAPILRADLAGIL
GVSGVLLFGCVYLLHLLRRHKHRPAPRLQPSRTSPQAPRARAWAPSQASQAALHVPY
ATINTSCRPATLDTAHPHGGPSWWASLPTHAAHRPQGPAAWASTPIPARGSFVSVENGL
YAQAGERPPHTGPGLTLFPDPRGPRAMEGPLGVR (SEQ ID NO: 45)

Human PVRIG extracellular segment amino acid sequence: (41-172)

TPEVWVQVRMEATELSSFTIRCGFLGSGSISLVTVSWGGPNGAGGTTLAVLHPERGIR
QWAPARQARWETQSSISLILEGSGASSPCANTTFCCKFASFPEGSWEACGSLPPSSDPG
LSAPPTPAPILRADL (SEQ ID NO: 46)

Human IgG1 constant region amino acid sequence:






 (SEQ ID NO: 47)

In a similar manner, a recombinant human CD112-human IgG1 Fc fusion protein was prepared. The full-length amino acid sequence of human CD112 was set forth in SEQ ID NO: 48, and the amino acid sequence of the extracellular segment of human CD112 was set forth in SEQ ID NO: 49.

Human CD112 full length amino acid sequence:

MARAAALLPSRSPPTPLLWPLLLLLLLETGAQDVRVQVLPEVRGQLGGTVELPCHLLP
PVPGLYISLVTWQRPDAPANHQNVAAFHPKMGPSFPSPKPGSERLSFVSAKQSTGQDT
EAELQDATLALHGLTVEDEGNYTCEFATFPKGSVRGMTWLRVIAKPKNQAEAQKVTF
SQDPTTVALCISKEGRPPARISWLSSLDWEAKETQVSGTLAGTVTVTSRFTLVPSGRAD
GVTVTCKVEHESFEEPALIPVTLSVRYPPEVSISGYDDNWYLGRTDATLSCDVRSNPEP
TGYDWSTTSGTFPTSAVAQGSQLVIHAVDSLFNTTFVCTVTNAVGMGRAEQVIFVRET
PNTAGAGATGGIIGGIIAAIIATAVAATGILICRQQRKEQTLQGAEEDEDLEGPPSYKPPT
PKAKLEAQEMPSQLFTLGASEHSPLKTPYFDAGASCTEQEMPRYHELPTLEERSGPLH
PGATSLGSPIPVPPGPPAVEDVSLDLEDEEGEEEEEYLDKINPIYDALSYSSPSDSYQGK
GFVMSRAMYV (SEQ ID NO: 48)

Human CD112 extracellular segment amino acid sequence: (32-360)

QDVRVQVLPEVRGQLGGTVELPCHLLPPVPGLYISLVTWQRPDAPANHQNVAAFHPK
MGPSFPSPKPGSERLSFVSAKQSTGQDTEAELQDATLALHGLTVEDEGNYTCEFATFP
KGSVRGMTWLRVIAKPKNQAEAQKVTFSQDPTTVALCISKEGRPPARISWLSSLDWE
AKETQVSGTLAGTVTVTSRFTLVPSGRADGVTVTCKVEHESFEEPALIPVTLSVRYPPE
VSISGYDDNWYLGRTDATLSCDVRSNPEPTGYDWSTTSGTFPTSAVAQGSQLVIHAVD
SLFNTTFVCTVTNAVGMGRAEQVIFVRETPNTAGAGATGG (SEQ ID NO: 49)

In a similar manner, a Macaca fascicularis PVRIG-human IgG1 Fc fusion protein was prepared. The Macaca fascicularis PVRIG amino acid sequence was set forth in SEQ ID NO: 50, and the Macaca fascicularis PVRIG extracellular segment sequence was set forth in SEQ ID NO: 51.

Macaca fascicularis PVRIG amino acid sequence:

MRTEAQVLALQSPEPGLEGAMGRRTLALPWVLLTLCVTAGTPEVWVQVQMEATELS
SFTVHCGFLGPGSISLVTVSWGGPDGAGGTKLAVLHPELGTRQWAPARQARWETQSSI
SLALEDSGASSPFANTTFCCKFASFPEGSWESCGSLPPSSDPGLSAPPTPVPILRADLAG

ILGLSGVLLFGCGYLLHLLRRQKHRPTPRLQPSHTNSQALTAQAWAPSQASQAALHDP
YATINTSFCPATLDTAHPNGWASLPTHAAHQPQGPAASASTPILARGSFVSVENGLYTQ
AGERPPHTGPGLTLFPDCRGPRALEGRFGVR (SEQ ID NO: 50)

Macaca fascicularis PVRIG extracellular segment sequence: (39-171)

AGTPEVWVQVQMEATELSSFTVHCGFLGPGSISLVTVSWGGPDGAGGTKLAVLHPEL
GTRQWAPARQARWETQSSISLALEDSGASSPFANTTFCCKFASFPEGSWESCGSLPPSS
DPGLSAPPTPVPILRAD (SEQ ID NO: 51)

### (2) Animal immunization, hybridoma cell fusion, and clone screening

The recombinant human PVRIG-human IgG1 Fc fusion protein obtained above was mixed sufficiently with an equal volume of complete Freund's adjuvant (CFA). The mixture was used to immunize BALB/c female mice aged 8-10 weeks (available from Shanghai SLAC Laboratory Animal Co., Ltd., with a body weigh of about 20g) for the first time, by intraperitoneal injection with 40 to 60 µg of the recombinant human PVRIG-human IgG1 Fc fusion protein per mouse; the immunization was carried out every 2 weeks, and the same dose of recombinant human PVRIG-human IgG1 Fc fusion protein was mixed with an equal volume of incomplete Freund's adjuvant; after immunization for a total of 5 times, when the mouse serum titer measured by ELISA was not less than 1: 105, 40 to 60 µg of the recombinant human PVRIG-human IgG1 Fc fusion protein was injected for boosting. On the third day after the boosting, splenocytes were isolated using standard techniques and fused with murine myeloma cells SP2/0 cells (ATCC accession number CRL-1581).

Four hybridoma cell lines were obtained by performing subclone screening on hybridoma cells that showed positive signals as identified by both ELISA and flow cytometry.

### (3) Expression and purification of monoclonal antibodies

Monoclonal antibodies were prepared by intraperitoneal inoculation of mice; female BALB/c mice aged 8-10 weeks were immunized intraperitoneally with 500 µl of sterile liquid paraffin; one week later, 1×10⁶ hybridoma cells were injected intraperitoneally; about 7-10 days later, ascites was harvested, and the supernatant was harvested by high-speed centrifugation. The supernatant obtained by the above method was purified by Protein G affinity chromatography to obtain monoclonal antibodies with a purity higher than 95%.

### (II) Variable region sequences of anti-PVRIG monoclonal antibodies

Candidate hybridoma cells were cultured to a total number of 10⁶, and the cells were harvested by centrifugation at 800 rpm for 10 min. Total RNA was extracted by Trizol kit (Invitrogen) and used as a template to synthesize cDNA library (Invitrogen) by reverse transcription. The variable region nucleic acid sequence corresponding to the hybridoma cells was amplified by PCR using cDNA as a template. Then the heavy chian and light chain variable region amino acid sequences (including CDR sequences) of the hybridoma cells were obtained by sequencing.

The amino acid sequences were identified, and the identified antibody sequences were respectively as follows:
The heavy chain variable region sequence of Antibody 4 was set forth in SEQ ID NO: 1, and the light chain variable region sequence of Antibody 4 was set forth in SEQ ID NO: 2; the heavy chain variable region sequence of Antibody 1 was set forth in SEQ ID NO: 39, and the light chain variable region sequence of Antibody 1 was set forth in SEQ ID NO: 40; the heavy chain variable region sequence of Antibody 2 was set forth in SEQ ID NO: 41, and the light chain variable region sequence of Antibody 2 was set forth in SEQ ID NO: 42; the heavy chain variable region sequence of Antibody 3 was set forth in SEQ ID NO: 43, and the light chain variable region sequence of Antibody 3 was set forth in SEQ ID NO: 44.

The above-mentioned antibodies numbered as Antibody 1, Antibody 2, Antibody 3, and Antibody 4 were all murine antibodies.

### (III) Expression and purification of humanized antibodies:

The various antibodies described above may be humanized. With Antibody 4 as an example, the resulting humanized antibodies include, but are not limited to antibodies 4-hu1 to 4-hu11, and may be prepared and purified generally as follows. The vector containing the HC and LC nucleic acid sequences encoding the humanized antibodies was obtained by artificial gene synthesis, suitable host cells such as HEK293 or CHO were used, and transient or stable transfection was performed using an expression system capable of antibody secretion with a optimal pre-set HC: LC vector ratio (e.g, 1:1 or 1:2). Subsequently, the cell culture supernatant was harvested and purified by Protein A affinity chromatography to obtain humanized antibodies with a purity of greater than 95%.

### Example 2

In Example 2, the different antibodies prepared in Example 1 above were evaluated for activity and antigen binding specificity. The isotype control used was mouse IgG1, κ (available from Biolegend).

The activity of the murine antibodies from different clones in binding to the human PVRIG-Fc fusion protein was evaluated by ELISA. The experimental procedure was as follows:

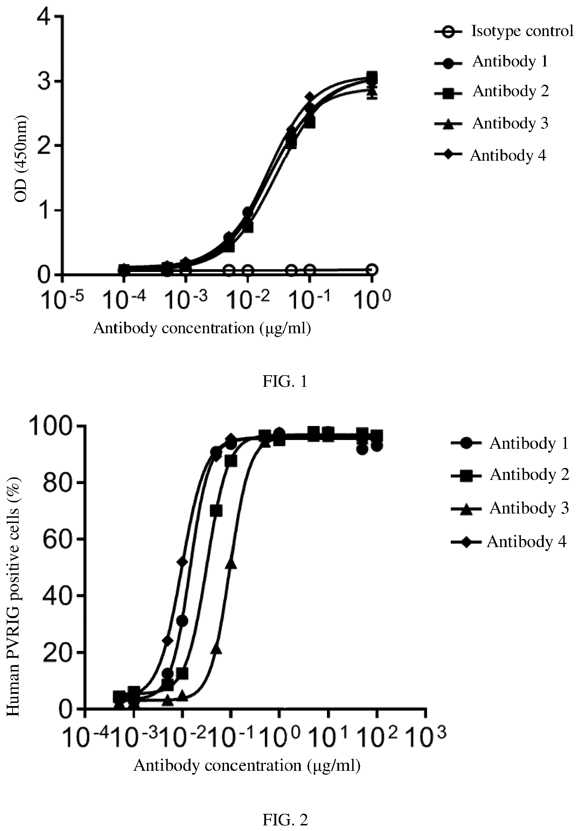

The recombinant human PVRIG-human IgG1 Fc fusion protein was diluted to 1.0 µg/ml in 1×PBS (phosphate buffer), the dilution was plated onto a 96-well ELISA plate overnight at 4°C by 100 µl/well, the plate was washed 3 times with PBST (0.05% Tween20-PBS) and blocked with 1% BSA, followed by incubation at 37°C for 2 h. The plate was washed 3 times with PBST, and murine antibodies (Antibody 1, Antibody 2, Antibody 3, or Antibody 4) diluted in multiple ratios were respectively added to set 8 concentrations, and the mixture was incubated at 37°C for 1 h using mouse IgG1 (available from Biolegend) as a negative control at 100 µl/well. The plate was washed 3 times with PBST, and 100 µl of horseradish peroxidase (HRP)-labeled goat anti-mouse IgG antibody (available from BOSTER, 1: 10,000 dilution) was added to each well, and the mixture was incubated at 37°C for 1 h. After washing, 100 µl of TMB substrate solution was added to each well for developing for 10 to 15 min in the dark, 100 µl of stop solution (2M H₂SO₄) was added to each well, the solution was measured with a microplate reader immediately after the reaction stopped, and the absorbance at 450 nm (OD450) was read. The results were shown in FIG. 1.

As can be seen from FIG. 1, all the murine antibodies from different clones were all able to specifically bind to the human PVRIG-Fc fusion protein.

The activity of murine antibodies from different clones in binding to human PVRIG protein on the cell membrane surface was evaluated by flow cytometry. The experimental procedure was as follows:
The 293T-human PVRIG cell line was harvested, washed with 1×PBS and resuspended, the cells were counted. Then 2×10⁵ cells were placed into each 1.5 ml Ep tube, the respective murine antibody (Antibody 1, Antibody 2, Antibody 3, or Antibody 4) was added at different concentrations, the mixture was incubated at 4°C for 30 min, then washed with 1×PBS, and centrifuged at 3,500 rpm for 5 min to harvest the cells. The cell pellet was resuspended in 100 µl of 1× PBS, a AF647-labeled goat anti-mouse IgG antibody (available from Biolegend) was added, the mixture was incubated at 4°C for 30 min in the dark, followed by washing twice with 1× PBS and harvesting the cells by centrifugation at 3,500 rpm for 5 min. The cell pellet was resuspended with 200 µl of l×PBS and measured using a flow cytometer (BD LSR II).

As can be seen from the results presented in FIG. 2, the murine antibodies from different clones were all able to specifically bind to the human PVRIG protein on the cell membrane surface.

The activity of murine antibodies from different clones in blocking the binding of the human PVRIG to its ligand CD112 was evaluated by flow cytometry. The experimental procedure was as follows:
The 293T-human CD112 cell line was harvested, and washed resuspended with 1×PBS to count the cells. Then 2×10⁵ cells were placed into each 1.5 ml Ep tube, the respective murine antibody (Antibody 1, Antibody 2, Antibody 3, or Antibody 4) at different concentrations and 10 µg/ml human CD112-human IgG1 Fc fusion protein were added, and the mixture was incubated at 4°C for 30 min, then added with 1×PBS, and centrifuged at 3,500 rpm for 5 min to harvest the cells. The cell pellet was resuspended in 100 µl of 1× PBS, a AF647-labeled mouse anti-human IgG-Fc antibody (available from Biolegend) was added, the mixture was incubated at 4°C for 30 min in the dark, followed by washing twice with 1× PBS and harvesting the cells by centrifugation at 3,500 rpm for 5 min. The cell pellet was resuspended with 200 ul of l× PBS and measured using a flow cytometer (BD LSR II).

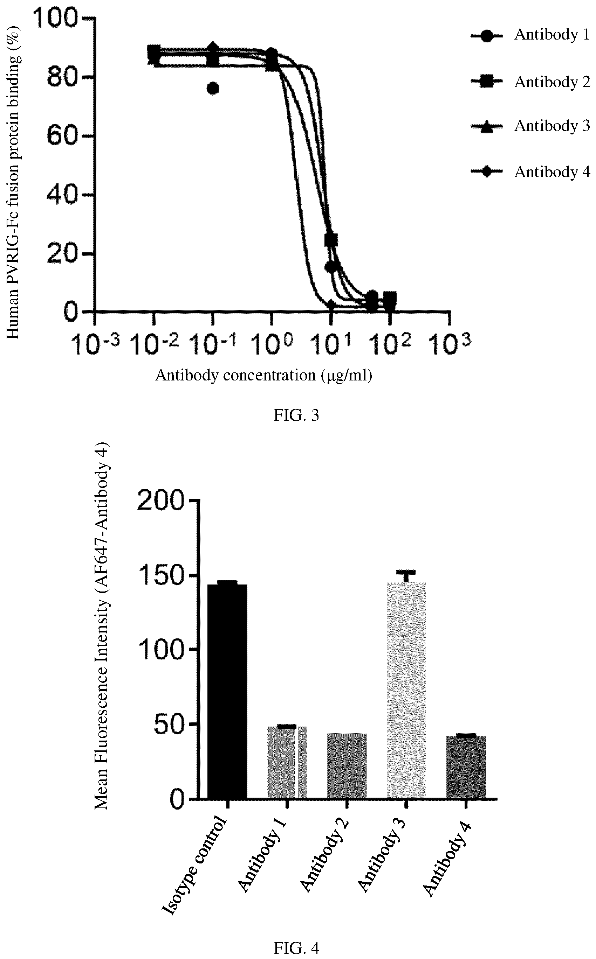

As can be seen from the results in FIG. 3, the murine antibodies from different clones were all able to effectively block the binding of human PVRIG to its ligand CD112.

The activity of murine antibodies from different clones in competitively binding to human PVRIG protein was evaluated by flow cytometry. The experimental procedure was as follows:
The 293T-human PVRIG cell line was harvested, and washed and resuspended with l×PBS to count the cells. Then 2×10⁵ cells were placed into each 1.5 ml Ep tube, 10 µg/ml control antibody mouse IgG1 and the murine antibody (Antibody 1, Antibody 2, Antibody 3, or Antibody 4) from different clones were added respectively, the mixture was incubated at 4°C for 30 min, then washed with 1×PBS, and centrifuged at 3,500 rpm for 5 min to harvest the cells. The cell pellet was resuspended in 100 µl of 1× PBS, a AF647-labeled Antibody 4 was added, the mixture was incubated at 4°C for 30 min in the dark, followed by washing twice with 1× PBS and harvesting the cells by centrifugation at 3,500 rpm for 5 min. The cell pellet was resuspended with 200 µl of l×PBS and measured using a flow cytometer (BD LSR II), and the analysis statistical results were shown in FIG. 4.

As can be seen in FIG. 4, Antibody 1, Antibody 2, and Antibody 4 have a relationship in competitively binding to human PVRIG, while Antibody 3 and Antibody 4 bind to different human PVRIG epitopes.

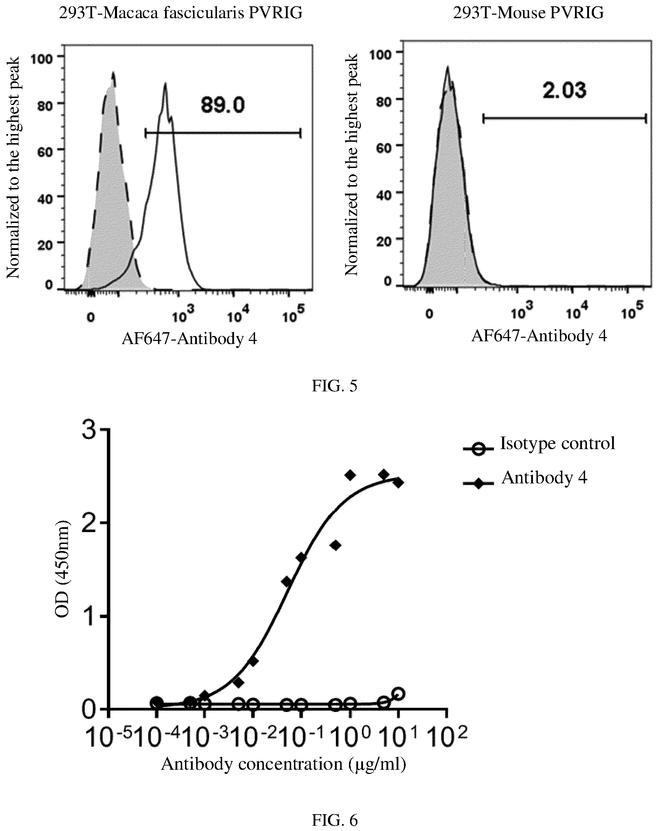

The activity of murine antibodies in binding to Macaca fascicularis PVRIG and mouse PVRIG was evaluated by flow cytometry. The experimental procedure was as follows:
The 293T-Macaca fascicularis PVRIG and 293T-mouse PVRIG cell lines were harvested, and washed and resuspended with 1× PBS to count the cells. Then 2×10⁵ cells were placed into each 1.5 ml Ep tube, a AF647-labeled Antibody 4 (a murine antibody) was added, the mixture was incubated at 4°C for 30 min in the dark, followed by washing twice with 1× PBS and harvesting the cells by centrifugation at 3,500 rpm for 5 min. The cell pellet was resuspended with 200 µl of l×PBS and measured using a flow cytometer (BD LSR II) and the analysis statistical results were shown in FIG. 5.

As can be seen from FIG. 5, Antibody 4 (a murine antibody) was able to bind to PVRIG protein from Macaca fascicularis but was not able to bind to PVRIG protein from mouse.

The activity of the murine antibody in binding to the Macaca fascicularis PVRIG was evaluated by an ELISA method. The experimental procedure was as follows:
The recombinant Macaca fascicularis PVRIG-human IgG1 Fc fusion protein was diluted to 1.0 µg/ml in 1×PBS (phosphate buffer), the dilution was placed in a 96-well ELISA plate overnight at 4°C by 100 µl/well, the plate was washed 3 times with PBST (0.05% Tween20-PBS) and blocked with 1% BSA, and the mixture was incubated for 2 h at 37°C. The plate was washed 3 times with PBST, and the murine antibody (Antibody 4) diluted in multiple ratios were respectively added to set 11 concentrations, and the mixture was incubated at 37°C for 1 h using mouse IgG1 as a negative control at 100 µl/well. The plate was washed 3 times with PBST, 100 µl of horseradish peroxidase (HRP)-labeled goat anti-mouse IgG antibody (available from BOSTER, 1: 10,000 dilution) was added to each well, and the mixture was incubated at 37°C for 1 h. After washing, 100 µl of TMB substrate solution was added to each well for developing for 10 to 15 min in the dark, 100 µl of stop solution (2M H₂SO₄) was added to each well, the solution was measured with a microplate reader immediately after the reaction stopped, and the absorbance at 450 nm (OD450) was read. The results were shown in FIG. 6. It can be seen that Antibody 4, a murine antibody, was able to bind to the PVRIG protein of Macaca fascicularis.

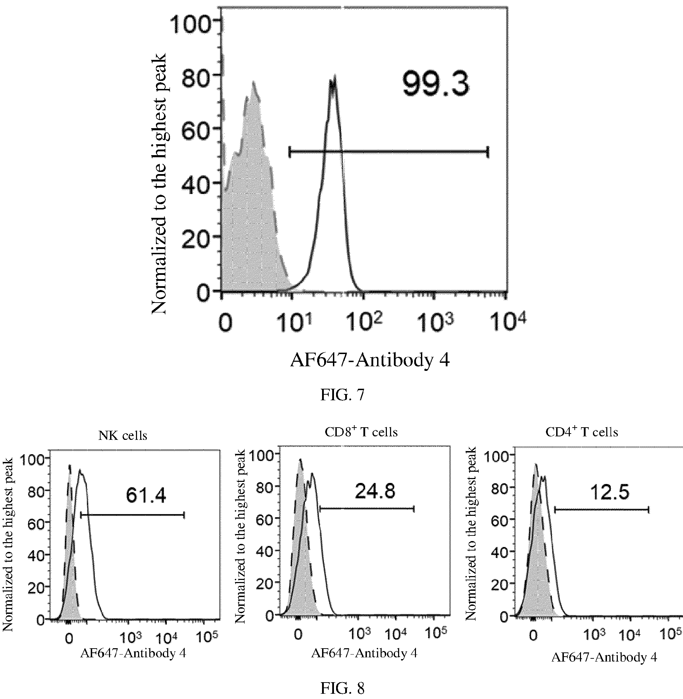

The activity of murine antibody in binding to PVRIG on the membrane surface of NKG cell line was evaluated by flow cytometry. The experimental procedure was as follows:
The NKG cell line was harvested, and washed and resuspended with l×PBS to count the cells. Then 2×10⁵ cells were placed into each 1.5 ml Ep tube, 10 µl of mouse serum was added to block at 4°C for 30 min, followed by washing with 1× PBS and harvesting the cells by centrifugation at 3,500 rpm for 5 min. The cell pellet was resuspended in 100 µl of 1× PBS, a AF647-labeled Antibody 4 was added, the mixture was incubated at 4°C for 30 min in the dark, followed by washing twice with 1× PBS and harvesting the cells by centrifugation at 3,500 rpm for 5 min. The cell pellet was resuspended with 200 µl of l×PBS and measured using a flow cytometer (BD LSR II). The measured results were shown in FIG. 7.

As can be seen from FIG. 7, murine Antibody 4 was able to bind to human PVRIG protein on the surface of the NKG cell line.

The activity of murine antibody in binding to PVRIG on the lymphocyte membrane surface was evaluated by flow cytometry. The experimental procedure was as follows:
Lymphocytes were isolated from normal human peripheral blood by centrifugation using Ficoll (Peripheral Blood Lymphocyte Separation Medium, GE Healthcare) at 400g for 30 min, and resuspended with l×PBS to count the cells. Then 1×10⁶ cells were placed into each 1.5 ml Ep tube, 10 µl of mouse serum was added to block at 4°C for 30 min, followed by washing twice with 1× PBS and harvesting the cells by centrifugation at 3,500 rpm for 5 min. The cell pellet was resuspended in 100 µl of 1×PBS, and AF647-labeled Antibody 4, PercP-Cy5.5-labeled mouse anti-human CD3 antibody (available from Biolegend), PE-Cy7-labeled mouse anti-human CD8 antibody (available from BD), BV421-labeled mouse anti-human CD4 antibody (available from BD), and BV605-labeled mouse anti-human CD56 antibody (available from Biolegend) were added. The mixture was incubated for 30 min at 4°C in the dark, followed by washing twice with 1×PBS and harvesting the cells by centrifugation at 3,500 rpm for 5 min. The cell pellet was resuspended with 200 µl of l×PBS and measured using a flow cytometer (BD LSR II). The measured results were shown in FIG. 8.

As can be seen from FIG. 8, murine Antibody 4 was able bind to the PVRIG protein on the human lymphocyte membrane surface.

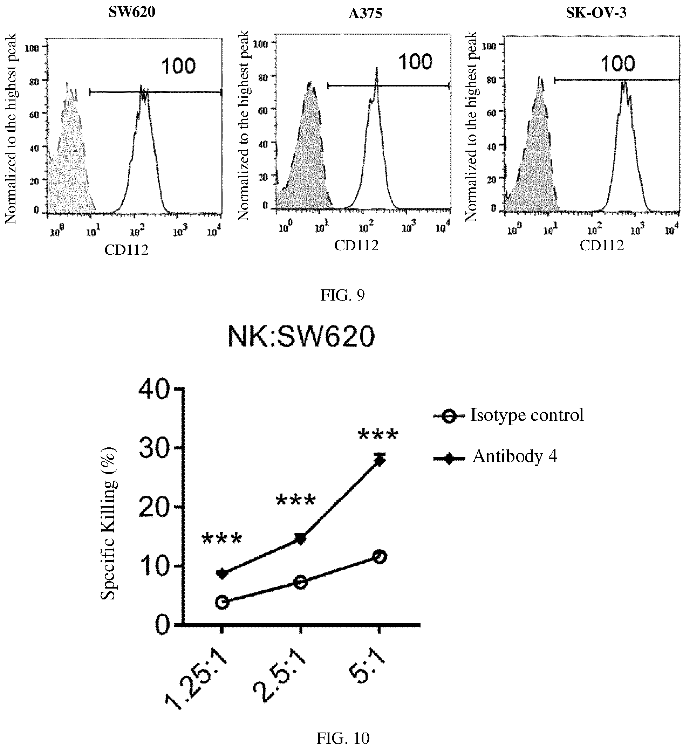

The expression of CD112 on the surface of tumor cell lines was evaluated by flow cytometry. The experimental procedure was as follows:
SW620 colon cancer cell line (Shanghai Cell Bank of Chinese Academy of Sciences), A375 melanoma cell line (Shanghai Cell Bank of Chinese Academy of Sciences), and SK-OV-3 ovarian cancer cell line (Shanghai Cell Bank of Chinese Academy of Sciences) were collected, and washed and resuspended with 1 × PBS to count the cells. Then 2×10⁵ cells were placed into each 1.5 ml Ep tube, 10 µl of mouse serum was added to block at 4°C for 30 min, followed by washing with 1× PBS and harvesting the cells by centrifugation at 3,500 rpm for 5 min. The cell pellet was resuspended in 100 µl of 1× PBS, a APC-labeled mouse anti-human CD112 antibody (available from Biolegend) was added, the mixture was incubated at 4°C for 30 min in the dark, followed by washing twice with 1× PBS and harvesting the cells by centrifugation at 3,500 rpm for 5 min. The cell pellet was resuspended with 200 µl of l×PBS and measured using a flow cytometer (BD LSR II). The measured results were shown in FIG. 9.

As can be seen from FIG. 9, the ligand CD112 of PVRIG was highly expressed on the surface of a variety of tumor cell lines.

A further experiment was conducted to evaluate the effect of murine antibodies on promoting the cytotoxicity of NK cells. The experimental procedure was as follows:
Lymphocytes were isolated from normal human peripheral blood by centrifugation using Ficoll (peripheral blood lymphocyte separation medium, GE Healthcare) at 400g for 30 min and resuspended with l×PBS; NK cells were subsequently isolated and purified using the NK cell isolation kit (Miltenyi Biotec, Human NK Cell Isolation Kit). Subsequently, the purified NK cells as effector cells and CFSE (available from Thermo Scientific)-labeled SW620 colon cancer cell line as target cells were placed into a 96-well round bottom plate at effector-to-target ratios of 1.25:1, 2.5:1, and 5:1, 10 µg/ml of mouse IgG1 as control antibody and a murine antibody (Antibody 4) were respectively added to the culture system, and the mixture was centrifuged at 250g for 4 min, and then incubated at 37°C for 4 h. The killed tumor cells were labeled with 7AAD and measured using a flow cytometer (BD LSR II) and the analysis statistical results were shown in FIG. 10.

As can be seen from FIG. 10, the murine antibody (Antibody 4) was able to greatly enhance the effect of NK cells on killing tumor cells at different effector-to-target ratios.

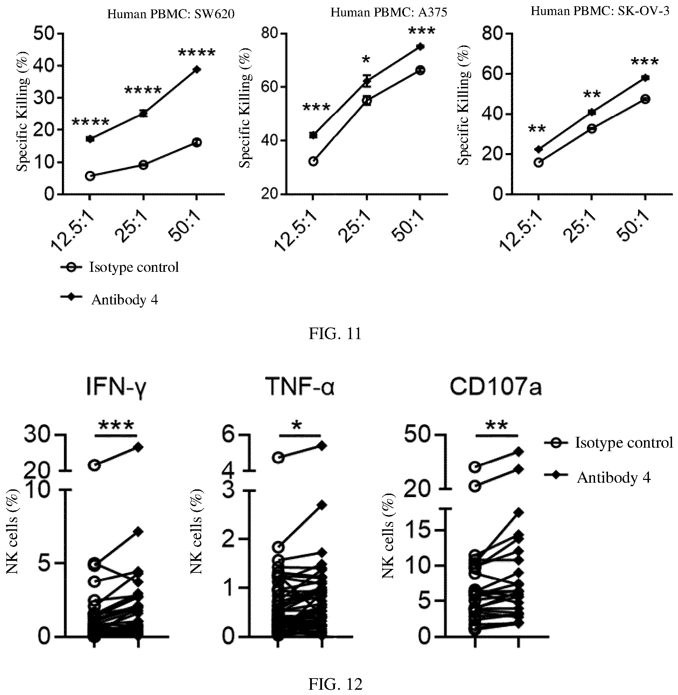

The effect of murine antibodies on promoting cytotoxicity of human PBMC was further evaluated. The experimental procedure was as follows:
Lymphocytes were isolated from normal human peripheral blood by centrifugation using Ficoll (peripheral blood lymphocyte separation medium, GE Healthcare) at 400g for 30 min and resuspended with l×PBS; subsequently, the human PBMC cells as effector cells and CFSE (available from Thermo Scientific)-labeled SW620 colon cancer cell line or A375 melanoma cells or SK-OV-3 ovarian cancer cells as target cells were placed into a 96-well round bottom plate at effector-to-target ratios of 12.5:1, 25:1, and 50:1, 10 µg/ml of mouse IgG1 as control antibody and a murine antibody (Antibody 4) were respectively added to the culture system, and the mixture was centrifuged at 250g for 4 min, and then incubated at 37°C for 4 h. The killed tumor cells were labeled with 7AAD and measured using a flow cytometer (BD LSR II) and the analysis statistical results were shown in FIG. 11.

As can be seen from FIG. 11, the murine antibody Antibody 4 was able to greatly enhance the effect of human PBMC cells on killing a variety of tumor cells at different effector-to-target ratios.

Flow cytometry was further used in the experiment to evaluate the effect of the murine antibody on promoting the expression of the effector molecules of the NK cells. The experimental procedure was as follows:
Lymphocytes were isolated from normal human peripheral blood by centrifugation using Ficoll (peripheral blood lymphocyte separation medium, GE Healthcare) at 400g for 30 min and resuspended with l×PBS; subsequently, the human PBMC cells as effector cells and SW620 colon cancer cell line as target cells were placed into a 96-well round bottom plate at an effector-to-target ratio of 25:1, 10 µg/ml of mouse IgG1 as control antibody and a murine antibody (Antibody 4) were respectively added to the culture system, and the mixture was centrifuged at 250g for 4 min, and then incubated at 37°C for 24 h. 2.5 µg/ml monensin (available from Sigma) and BV510-labeled mouse anti-human CD107a antibody (available from Biolegend) were added during the last 4 h of incubation. At the end of the incubation, the cells were harvested, washed with 1×PBS, and resuspended in 100 µl of 1×PBS, and 10 µl of mouse serum was added to block for 30 min at 4°C. The cells were washed with 1× PBS and resuspended in 100 µl of 1×PBS, and PercP-Cy5.5-labeled mouse anti-human CD3 antibody (available from Biolegend), PE-Cy7-labeled mouse anti-human CD8 antibody (available from BD), BV421-labeled mouse anti-human CD4 antibody (available from BD), and BV605-labeled mouse anti-human CD56 antibody (available from Biolegend) were added respectively, the mixture was incubated for 30 min at 4°C in the dark, followed by washing twice with 1×PBS and harvesting the cells by centrifugation at 350g for 5 min. The cells were resuspended in Foxp3/Transcription Factor Staining Buffer (available from eBioscience), and FITC-labeled mouse anti-human IFN-γ (available from Biolegend) and PE-labeled mouse anti-human TNF-α (available from Biolegend) were added and incubated for 1 h at 4°C. Then the cells were washed twice with l×PBS and harvested by centrifugation at 500g for 5 min. The cells were resuspended in 200 µl of l×PBS and measured using a flow cytometer (BD FACS Celesta) and the analysis statistical results were shown in FIG. 12.

As can be seen from FIG. 12, the murine antibody (Antibody 4) was able to enhance the degranulation capacity of NK cells and promote the secretion of cytokines.

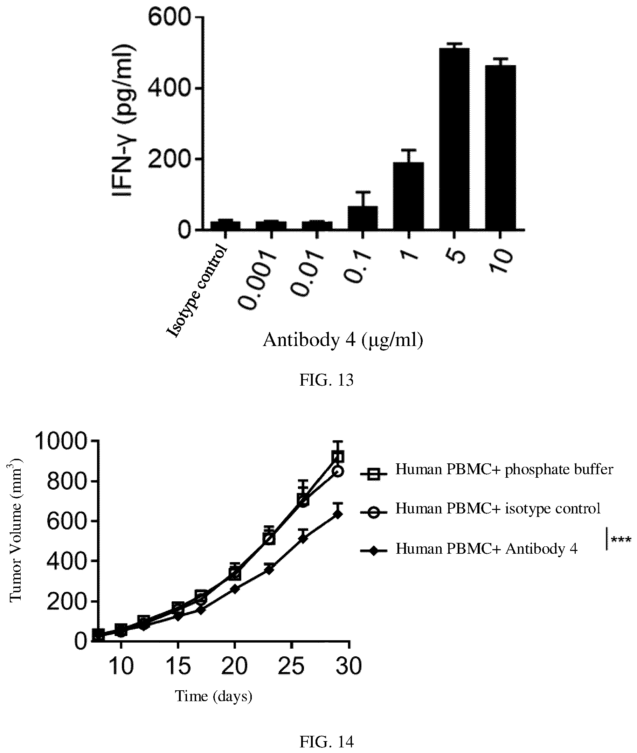

The effect of murine antibodies on promoting cytokine secretion of NK cell was evaluated by ELISA in the experiments. The experimental procedure was as follows: lymphocytes were isolated from normal human peripheral blood by centrifugation using Ficoll (peripheral blood lymphocyte separation medium, GE Healthcare) at 400g for 30 min and resuspended with l×PBS. NK cells were subsequently isolated and purified using the NK cell isolation kit (Miltenyi Biotec, Human NK Cell Isolation Kit). Subsequently, the purified NK cells as effector cells and SW620 colon cancer cell line as target cells were placed into a 96-well round bottom plate at an effector-to-target ratio of 2.5:1, 10 µg/ml mouse IgG1 as control antibody and a murine antibody (Antibody 4) were respectively added to the culture system, and the mixture was centrifuged at 250g for 4 min, and then incubated at 37°C for 18 h. The cell culture supernatant was then harvested and the content of IFN-γ in the culture supernatant was measured by ELISA kit (available from Dakewe). The ELISA analysis statistical results were shown in FIG. 13. It can be seen that the murine antibody (Antibody 4) was able to promot the secretion of IFN-γ by NK cells.

The activity of the murine antibody in inhibiting tumor growth was evaluated in the experiment. The experimental procedure was as follows:
SW620 colon cancer cells (Shanghai Cell Bank of Chinese Academy of Sciences) were amplified in vitro, then harvested and washed twice with 1×PBS; the cells were harvested by centrifugation at 800 rpm for 5 min, resuspended with sterile normal saline for cell counting; the cell concentration was adjusted to 1×10⁷ cells/ml; 100 µl of the cells were inoculated subcutaneously in the right armpit of 6-8 week old B-NDG mice; on day 7, the tumor-bearing mice were randomly divided into groups, and the human PBMC cells isolated by Ficoll were transfused via tail vein, with 1×10⁷ cells per mouse. Intraperitoneal injection of PBS or mouse IgG control antibody or murine antibody Antibody 4 (250 µg/animal) was started the next day, once every 3 days for a total of 5 times of treatment. The long and short sides of the subcutaneous tumor of the mice were measured every 3 days and the mice were weighed. Tumor size was calculated according to the empirical formula (tumor size = long side x short side x short side/2). The results were shown in FIG. 14. The results showed that the murine antibody (Antibody 4) was able to inhibit the growth of subcutaneous tumors in vivo.

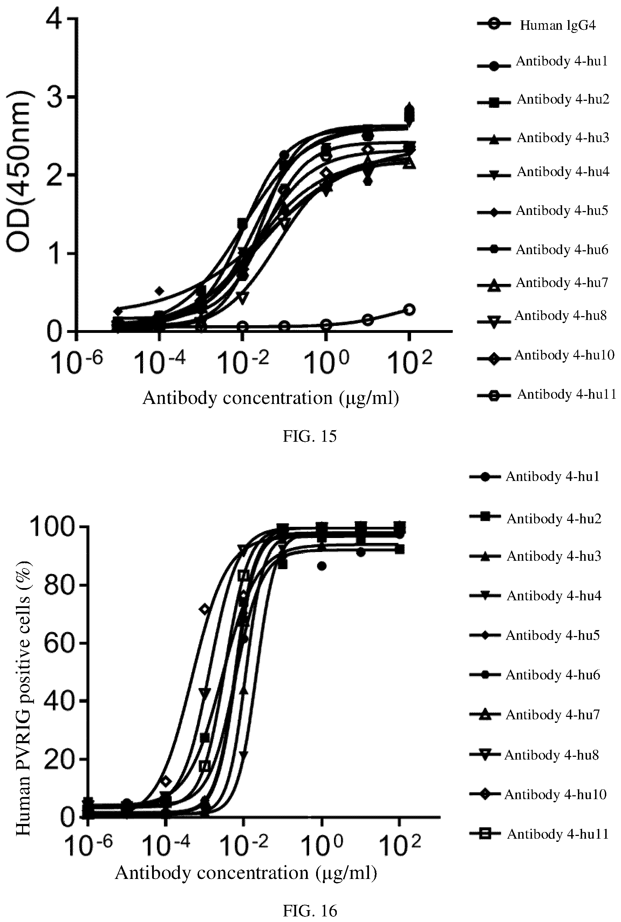

The activity of the humanized antibody in binding to human PVRIG was further evaluated by ELISA in the experiment. The experimental procedure was as follows:
The recombinant human PVRIG-human IgG1 Fc fusion protein was diluted to 1.0 µg/ml in 1×PBS (phosphate buffer), the dilution was plated onto a 96-well ELISA plate overnight at 4°C by 100 µl/well, the plate was washed 3 times with PBST (0.05% Tween20-PBS) and blocked with 1% BSA, and the mixture was incubated for 2 h at 37°C. The plate was washed 3 times with PBST, and humanized antibodies diluted in multiple ratios were respectively added to set 8 concentrations, and the mixture was incubated at 37°C for 1 h using human IgG4 as a negative control at 100 µl/well. The plate was washed 3 times with PBST, and 100 µl of horseradish peroxidase (HRP)-labeled mouse anti-human IgG4-Fc antibody (available from Southern Biotech, 1: 5,000 dilution) was added to each well, and the mixture was incubated at 37°C for 1 h. After washing, 100 µl of TMB substrate solution was added to each well for developing for 10 to 15 min in the dark, 100 µl of stop solution (2M H₂SO₄) was added to each well, the solution was measured with a microplate reader immediately after the reaction stopped, and the absorbance at 450 nm (OD450) was read. The results were shown in FIG. 15.

As can be seen from FIG. 15, all the humanized antibodies were able to specifically bind to the human PVRIG protein.

The activity of the humanized antibodies in binding to human PVRIG was evaluated by flow cytometry in the experiment.

The 293T-human PVRIG cell line was harvested, and washed and resuspended with l×PBS to count the cells. Then 2×10⁵ cells were placed into each 1.5 ml Ep tube, the respective humanized antibody was added at different concentrations, the mixture was incubated at 4°C for 30 min, then washed with 1×PBS, and centrifuged at 3,500 rpm for 5 min to harvest the cells. The cell pellet was resuspended in 100 µl of 1× PBS, a AF647-labeled mouse anti-human IgG-Fc antibody (available from Biolegend) was added, the mixture was incubated at 4°C for 30 min in the dark, followed by washing twice with 1× PBS and harvesting the cells by centrifugation at 3,500 rpm for 5 min. The cell pellet was resuspended with 200 µl of l×PBS and measured using a flow cytometer (BD LSR II), and the analysis statistical results were shown in FIG. 16. It can be seen that all the humanized antibodies were ablet to specifically bind to the human PVRIG protein on the cell membrane surface.

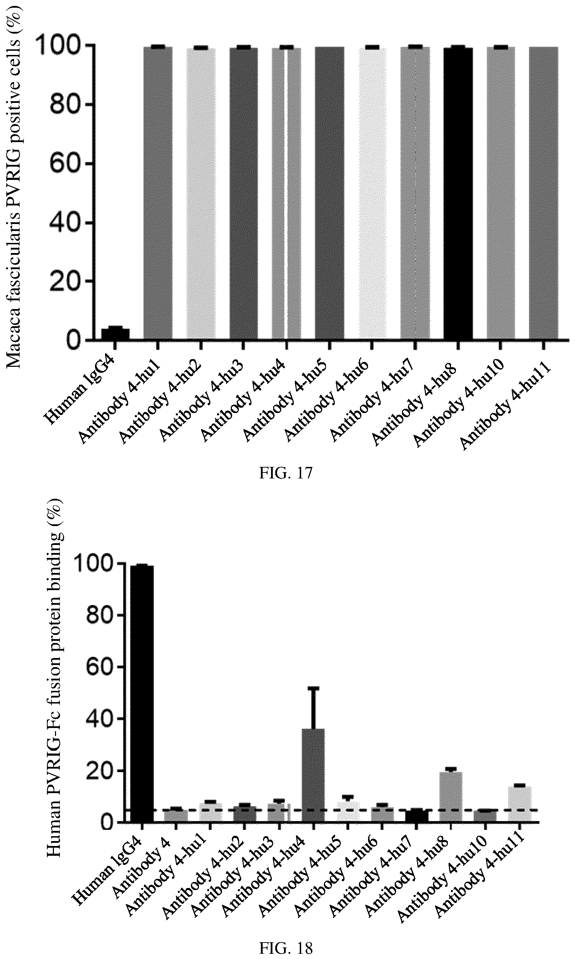

The activity of the humanized antibody in binding to Macaca fascicularis PVRIG was evaluated by flow cytometry in the experiment. The experimental procedure was as follows:
The 293T-Macaca fascicularis PVRIG cell line was harvested, and washed and resuspended with 1× PBS to count the cells. Then 2×10⁵ cells were placed into each 1.5 ml Ep tube, the respective humanized antibody was added at different concentrations, the mixture was incubated at 4°C for 30 min, then washed with 1×PBS, and centrifuged at 3,500 rpm for 5 min to harvest the cells. The cell pellet was resuspended in 100 µl of 1× PBS, a AF647-labeled mouse anti-human IgG-Fc antibody (available from Biolegend) was added, the mixture was incubated at 4°C for 30 min in the dark, followed by washing twice with 1× PBS and harvesting the cells by centrifugation at 3,500 rpm for 5 min. The cell pellet was resuspended with 200 µl of l×PBS and measured using a flow cytometer (BD LSR II), and the analysis statistical results were shown in FIG. 17.

As can be seen from FIG. 17, all the humanized antibodies were able to specifically bind to the Macaca fascicularis PVRIG protein on the cell membrane surface.

The activity of the humanized antibodies in blocking the binding of human PVRIG to ligand was evaluated by flow cytometry in the experiment. The experimental procedure was as follows:
The 293T-human CD112 cell line was harvested, and washed and resuspended with 1×PBS to count the cells. Then 2×10⁵ cells were placed into each 1.5 ml Ep tube, the humanized antibody and 10 µg/ml human PVRIG-human IgG1 Fc fusion protein were added, and the mixture was incubated at 4°C for 30 min, then washed with 1×PBS, and centrifuged at 3,500 rpm for 5 min to harvest the cells. The cell pellet was resuspended in 100 µl of 1× PBS, a AF647-labeled mouse anti-human IgG-Fc antibody (available from Biolegend) was added, the mixture was incubated at 4°C for 30 min in the dark, followed by washing twice with 1× PBS and harvesting the cells by centrifugation at 3,500 rpm for 5 min. The cell pellet was resuspended with 200 µl of l× PBS and measured using a flow cytometer (BD LSR II). As shown in FIG. 18, the analysis statistical results show that all the humanized antibodies were able to effectively blocking the binding of human PVRIG to its ligand CD112.

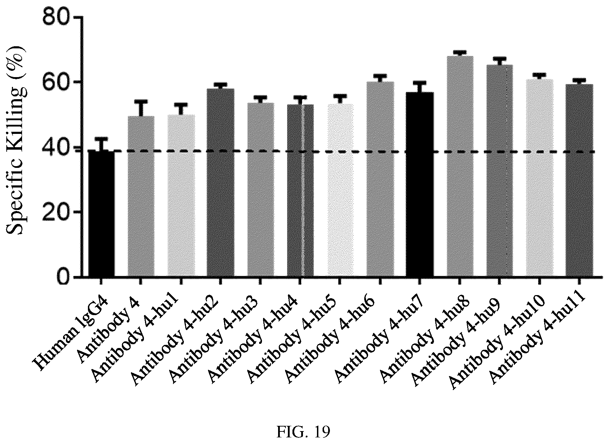

The effect of the humanized antibodies on promoting the cytotoxicity of human PBMC was further evaluated in the experiment. The experimental procedure was as follows:
Lymphocytes were isolated from normal human peripheral blood by centrifugation using Ficoll (peripheral blood lymphocyte separation medium, GE Healthcare) at 400g for 30 min and resuspended with l×PBS; subsequently, the human PBMC cells as effector cells and CFSE (available from Thermo Scientific)-labeled SW620 colon cancer cell line as target cells were placed into a 96-well round bottom plate at an effector-to-target ratio of 25:1, 10 µg/ml human IgG4 as control antibody, a murine antibody (Antibody 4) , and the humanized antibody were respectively added to the culture system, and the mixture was centrifuged at 250g for 4 min, and then incubated at 37°C for 4 h. The killed tumor cells were labeled with 7AAD and measured using a flow cytometer (BD LSR II) and the analysis statistical results were shown in FIG. 19. It can be seen that all the humanized antibodies were able to greatly promote the killing of tumor cells by human PBMC cells.

The affinity constants were determined by surface plasmon resonance (SPR):
The binding kinetics and dissociation equilibrium constant (KD) of the antibodies of the present disclosure to human PVRIG were determined using the surface plasmon resonance (Biacore) assay. Briefly, a CM5 chip was coated with human PVRIG-human IgG1 Fc fusion protein, and after being blocked, antibody solutions of different concentrations passed through the CM5 chip at a fixed flow rate and bound to antigen for 2 min, followed by transfer to dissociation buffer for 6 min for determination of the dissociation rate. The kinetics were analyzed using a 1:1 binding model.

In experiments substantially as described above, the determined affinity of the murine antibodies was as shown in Table 1, and the determined affinity of the humanized antibodies was as shown in Table 2.

**Table 1 Affinity of murine antibody**

| Clone | ka (1/Ms) | kd (1/s) | **KD(M)** |
|---|---|---|---|
| Antibody 1 | 1.33E+05 | 4.19E-05 | 3.16E-10 |
| Antibody 2 | 9.05E+04 | 1.37E-04 | 1.51E-09 |
| Antibody 3 | 8.91E+04 | 8.82E-05 | 9.90E-10 |
| Antibody 4 | 2.44E+05 | 1.00E-06 | 4.10E-12 |

**Table 2 Affinity of humanized antibody**

| Antibody | ka (1/Ms) | kd (1/s) | KD(M) |
|---|---|---|---|
| Antibody 4-hu1 | 1.97E+05 | 2.32E-05 | 1.18E-10 |
| Antibody 4-hu2 | 2.66E+05 | 6.38E-05 | 2.39E-10 |
| Antibody 4-hu3 | 2.12E+05 | 1.95E-05 | 9.16E-11 |
| Antibody 4-hu4 | 1.88E+05 | 4.44E-05 | 2.36E-10 |
| Antibody 4-hu5 | 2.36E+05 | 3.54E-06 | 1.50E-11 |
| Antibody 4-hu6 | 2.81E+05 | 1.00E-06 | 3.56E-12 |
| Antibody 4-hu7 | 2.77E+05 | 1.00E-06 | 3.61E-12 |
| Antibody 4-hu8 | 1.98E+05 | 1.00E-06 | 5.05E-12 |
| Antibody 4-hu10 | 2.50E+05 | 1.00E-06 | 4.00E-12 |
| Antibody 4-hu11 | 2.37E+05 | 1.00E-06 | 4.22E-12 |

It can be seen from the above that the obtained Antibodies 1 to 4 and the corresponding humanized antibodies of Antibody 4 all exhibited excellent affinity for PVRIG, and exhibited a good antitumor effect.

In addition, description with reference to the term "an embodiment", "some embodiments", "an example", "a specific example", or "some examples" or the like means that a specific feature, structure, material, or characteristic described in combination with the embodiment(s) or example(s) is included in at least one embodiment or example of the present disclosure. In this specification, illustrative expressions of these terms do not necessarily refer to the same embodiment or example. Moreover, the specific features, structures, materials, or characteristics described may be combined in any suitable manner in any one or more embodiments or examples. In addition, without mutual contradiction, those skilled in the art may incorporate and combine different embodiments or examples and features of the different embodiments or examples described in this specification.

Although the embodiments of the present disclosure have been illustrated and described, it should be understood that the above mebodiments are exemplary and should not be construed as limiting the present disclosure, and persons of ordinary skill in the art may make various changes, modifications, replacements and variations to the above embodiments without departing from the scope of the present disclosure.

## Claims

1. An antibody or antibody fragment comprising a heavy chain variable region and a light chain variable region, the heavy chain variable region being selected from the group consisting of sequences set forth in SEQ ID NO: 9, SEQ ID NO: 15, SEQ ID NO: 18, SEQ ID NO: 21, SEQ ID NO: 24, SEQ ID NO: 27, SEQ ID NO: 30, and SEQ ID NO: 33, and the light chain variable region being a sequence set forth in SEQ ID NO: 36.

2. The antibody or antibody fragment according to claim 1, comprising a heavy chain and a light chain,
wherein an amino acid sequence of the heavy chain is a sequence set forth in SEQ **ID** NO: 10, SEQ **ID** NO: 16, SEQ **ID** NO: 19, SEQ ID NO: 22, SEQ **ID** NO: 25, SEQ **ID** NO: 28, SEQ ID NO:31, or SEQ ID NO: 34; and
wherein an amino acid sequence of the light chain is a sequence set forth in SEQ ID NO: 37.

3. An isolatable nucleic acid, encoding the antibody or antibody fragment according to claim 1 or 2.

4. An expression vector, comprising the nucleic acid according to claim 3.

5. A host cell, expressing the antibody or antibody fragment according to claim 1 or 2.

6. The host cell according to claim 5, comprising the expression vector according to claim 4.

7. A pharmaceutical composition, comprising the antibody or antibody fragment according to claim 1 or 2 and a pharmaceutically acceptable carrier.

8. A pharmaceutical combination, comprising:
(1) the antibody or antibody fragment according to claim 1 or 2, or the pharmaceutical composition according to claim 7; and
(2) an antibody for cancer therapy other than (1).

9. A method for inhibiting PVRIG protein activity in a sample in vitro, comprising contacting the sample with the antibody or antibody fragment according to claim 1 or 2, or the pharmaceutical composition according to claim 7.

10. The antibody or antibody fragment according to any one of claims 1 to 2 or the pharmaceutical composition according to claim 7 for use in preventing or treating cancer.

## Patentansprüche

1. Antikörper oder Antikörperfragment, umfassend eine schwere Kettenvariable Region und eine leichte Kettenvariable Region, wobei die schwere Kettenvariable Region ausgewählt ist aus der Gruppe bestehend aus den gemäß SEQ ID NO: 9, SEQ ID NO: 15, SEQ ID NO: 18, SEQ ID NO: 21, SEQ ID NO: 24, SEQ ID NO: 27, SEQ ID NO: 30 und SEQ ID NO: 33 angegebenen Sequenzen, und die leichte Kettenvariable Region eine gemäß SEQ ID NO: 36 angegebene Sequenz ist.

2. Antikörper oder das Antikörperfragment nach Anspruch 1, umfassend eine schwere Kette und eine leichte Kette,
wobei eine Aminosäuresequenz der schweren Kette eine gemäß SEQ ID NO: 10, SEQ ID NO: 16, SEQ ID NO: 19, SEQ ID NO: 22, SEQ ID NO: 25, SEQ ID NO: 28, SEQ ID NO: 31 oder SEQ ID NO: 34 angegebene Sequenz ist; und
wobei eine Aminosäuresequenz der leichten Kette eine gemäß SEQ ID NO: 37 angegebene Sequenz ist.

3. Isolierbare Nukleinsäure, die den Antikörper oder das Antikörperfragment nach Anspruch 1 oder 2 kodiert.

4. Expressionsvektor, umfassend die Nukleinsäure nach Anspruch 3.

5. Wirtszelle, die den Antikörper oder das Antikörperfragment nach Anspruch 1 oder 2 exprimiert.

6. Wirtszelle nach Anspruch 5, umfassend den Expressionsvektor nach Anspruch 4.

7. Pharmazeutische Kombination, umfassend den Antikörper oder das Antikörperfragment nach Anspruch 1 oder 2 und einen pharmazeutisch verträglichen Träger.

8. Pharmazeutische Kombination, umfassend:
(1) den Antikörper oder das Antikörperfragment nach Anspruch 1 oder 2 oder die pharmazeutische Kombination nach Anspruch 7; und
(2) einen von (1) verschiedenen Antikörper zur Krebstherapie.

9. Verfahren zum Inhibieren der Aktivität des PVRIG-Proteins in einer Probe in vitro, umfassend das Inkontaktbringen der Probe mit dem Antikörper oder Antikörperfragment nach Anspruch 1 oder 2 oder der pharmazeutischen Kombination nach Anspruch 7.

10. Antikörper oder das Antikörperfragment nach einem der Ansprüche 1 bis 2 oder die pharmazeutische Kombination nach Anspruch 7 zur Verwendung bei der Prävention oder Behandlung von Krebs.

## Revendications

1. Un anticorps ou un fragment d'anticorps comprenant une région variable de chaîne lourde et une région variable de chaîne légère, la région variable de chaîne lourde étant sélectionnée dans le groupe constitué des séquences représentées par SEQ ID NO: 9, SEQ ID NO: 15, SEQ ID NO: 18, SEQ ID NO: 21, SEQ ID NO: 24, SEQ ID NO: 27, SEQ ID NO: 30 et SEQ ID NO: 33, et la région variable de chaîne légère étant la séquence représentée par SEQ ID NO: 36.

2. Anticorps ou fragment d'anticorps selon la revendication 1, comprend une chaîne lourde et une chaîne légère,
dans laquelle la séquence d'acides aminés de la chaîne lourde est la séquence représentée par SEQ ID NO: 10, SEQ ID NO: 16, SEQ ID NO: 19, SEQ ID NO: 22, SEQ ID NO: 25, SEQ ID NO: 28, SEQ ID NO: 31 ou SEQ ID NO: 34 ; et
dans laquelle la séquence d'acides aminés de la chaîne légère est la séquence représentée par SEQ ID NO: 37.

3. Acide nucléique isolable codant pour l'anticorps ou fragment d'anticorps selon la revendication 1 ou 2.

4. Vecteur d'expression, comprenant l'acide nucléique selon la revendication 3.

5. Cellule hôte exprimant l'anticorps ou fragment d'anticorps selon la revendication 1 ou 2.

6. Cellule hôte selon la revendication 5, comprenant le vecteur d'expression selon la revendication 4.

7. Composition pharmaceutique, comprenant l'anticorps ou le fragment d'anticorps selon la revendication 1 ou 2 et un véhicule pharmaceutiquement acceptable.

8. Combinaison pharmaceutique, comprenant :
(1) l'anticorps ou le fragment d'anticorps selon la revendication 1 ou 2, ou la composition pharmaceutique selon la revendication 7 ; et
(2) un anticorps de traitement du cancer différent de celui défini à (1).

9. Une méthode d'inhibition de l'activité de la protéine PVRIG dans un échantillon in vitro, comprenant la mise en contact de l'échantillon avec l'anticorps ou le fragment d'anticorps selon la revendication 1 ou 2, ou la composition pharmaceutique selon la revendication 7.

10. Anticorps ou fragment d'anticorps selon l'une quelconque des revendications 1 à 2, ou la composition pharmaceutique selon la revendication 7, pour utilisation dans la prévention ou le traitement du cancer.
